# EUROPEAN PATENT APPLICATION

(11) **EP 4 725 965 A1**
(43) Date of publication of application: **15.04.2026**
(21) Application number: 24822634.2
(22) Date of filing: 06.06.2024
(51) Int. Cl.: C07K 16/28, C07K 19/00, C12N 15/13, C12N 15/62, C12N 15/85, C12N 5/10, A61K 39/395, A61P 37/00, A61P 17/06, A61P 13/12, A61P 19/02, A61P 29/00, A61P 1/00, A61P 17/00, A61P 3/10, A61P 21/00

(54) **ANTI-BDCA2 ANTIBODY**

(30) Priority: 12.06.2023 CN 202310694536
(71) Applicant: Sunshine Guojian Pharmaceutical (Shanghai) Co., Ltd., Shanghai 201203 (CN)
(72) Inventor: HUANG, Haomin, Shanghai 201203 (CN); DENG, Lan, Shanghai 201203 (CN); LOU, Jing, Shanghai 201203 (CN)
(74) Representative: dompatent
(86) International application number: PCT/CN2024/097846
(87) International publication number: WO 2024/255683

(57) **Abstract**

Provided is an anti-BDCA2 antibody. The anti-BDCA2 antibody is capable of binding to the BDCA2 antigen with high specificity, exhibits high affinity, effectively inhibits CpG-A-stimulated secretion of IFNα and IgM by PBMCs, showing potential as a therapeutic agent for autoimmune diseases.

## Description

### TECHNICAL FIELD

The present invention relates to the field of antibodies. Specifically, it relates to an anti-BDCA2 antibody.

### BACKGROUND ART

Type I interferon (IFN-I) is an inflammatory mediator primarily produced by plasmacytoid dendritic cells (pDCs) and is a component of the innate immune system. Type I IFN genes are tightly regulated. Under normal circumstances, the formation of IFN-α is almost undetectable in healthy individuals. However, many patients with systemic autoimmune diseases exhibit signs of sustained Type I IFN production and increased expression of IFN-α-regulated genes. IFN-α activates both the innate and adaptive immune systems. When an individual possesses a genetic predisposition leading to robust IFN-α production and/or a pronounced IFN-α response, immune tolerance is disrupted, resulting in the generation of antibodies against self-antigens. These antibodies, together with self-antigens, form immune complexes (ICs) that stimulate pDCs to synthesize IFN-α and activate B cells to promote autoantibody production, thereby establishing a vicious cycle of sustained IFN-α production and autoimmune responses.

Blood dendritic cell antigen 2 (BDCA2), also designated as CLEC4C or CD303, is specifically expressed only on pDCs. Triggering of BDCA2 activates SYK protein, leading to the activation of a complex composed of BLNK, BTK, and PLCγ2, which subsequently mobilizes intracellular Ca²⁺ and inhibits the release of inflammatory mediators such as IFN-I and IL-6 mediated by TLR7 and TLR9 signaling pathways. BDCA2-targeting therapeutics may function by modulating pDC activity, facilitating rapid internalization of BDCA2 from the pDC surface, and indirectly suppressing the release of IFN-I and pro-inflammatory cytokines upstream. Furthermore, BDCA2 signaling is not influenced by other IFN-I-inducing stimuli, making BDCA2 targeting a novel therapeutic strategy for autoimmune diseases. However, existing BDCA2-targeting agents in the prior art still exhibit numerous limitations, indicating a persistent need in the art for novel anti-BDCA2 antibodies.

### SUMMARY OF THE INVENTION

An object of the present invention is to provide an anti-BDCA2 antibody.

In a first aspect of the invention, there is provided an antibody or antigen-binding fragment thereof that binds to BDCA2, characterized by comprising a heavy chain variable region and a light chain variable region, the heavy chain variable region comprising heavy chain complementarity determining regions H-CDR1, H-CDR2, and H-CDR3, wherein
the amino acid sequence of H-CDR1 is as shown in SYX₁IH (SEQ ID NO: 30),
the amino acid sequence of H-CDR2 is as shown in TIYPGX₂GDTSYNQKFKG (SEQ ID NO: 31),
the amino acid sequence of H-CDR3 is as shown in MGDNEYFDY (SEQ ID NO: 8), and
the light chain variable region comprises light chain complementarity determining regions L-CDR1, L-CDR2, and L-CDR3; wherein,
the amino acid sequence of L-CDR1 is as shown in RASGNIHNYLA (SEQ ID NO: 9),
the amino acid sequence of L-CDR2 is as shown in DAETLAX₃ (SEQ ID NO: 32),
the amino acid sequence of L-CDR3 is as shown in QHFWSTPYT (SEQ ID NO: 11);
wherein, X₁ is I or L; X₂ is an amino acid selected from the group consisting of: N, G, A, S, R; and/or X₃ is an amino acid selected from the group consisting of: D, I, or E.

In another preferred embodiment, X₁, X₂, X₃ are selected from the group consisting of:
X₁ is I, X₂ is N, X₃ is D;
X₁ is L, X₂ is N, X₃ is I;
X₁ is L, X₂ is N, X₃ is E;
X₁ is L, X₂ is G, X₃ is I;
X₁ is L, X₂ is G, X₃ is E;
X₁ is L, X₂ is R, X₃ is I;
X₁ is L, X₂ is R, X₃ is E;
X₁ is I, X₂ is G, X₃ is I;
X₁ is I, X₂ is G, X₃ is E;
X₁ is I, X₂ is R, X₃ is I; or
X₁ is I, X₂ is R, X₃ is E.

In another preferred embodiment, the heavy chain variable region comprises heavy chain complementarity determining regions H-CDR1, H-CDR2, and H-CDR3, wherein the amino acid sequence of H-CDR1 is as shown in SYIIH (SEQ ID NO: 6),
the amino acid sequence of H-CDR2 is as shown in TIYPGNGDTSYNQKFKG (SEQ ID NO: 7),
the amino acid sequence of H-CDR3 is as shown in MGDNEYFDY (SEQ ID NO: 8), and
the light chain variable region comprises light chain complementarity determining regions L-CDR1, L-CDR2, and L-CDR3; wherein,
the amino acid sequence of L-CDR1 is as shown in RASGNIHNYLA (SEQ ID NO: 9),
the amino acid sequence of L-CDR2 is as shown in DAETLAD (SEQ ID NO: 10),
the amino acid sequence of L-CDR3 is as shown in QHFWSTPYT (SEQ ID NO: 11);
wherein any one of the aforementioned amino acid sequences further optionally comprises a derivative sequence that optionally undergoes addition, deletion, modification, and/or substitution of at least one amino acid and is capable of retaining BDCA2 binding affinity.

In another preferred embodiment, the ratio (F1/F0) of the affinity F1 of the derivative antibody binding to BDCA2 to the affinity F0 of the corresponding non-derivative antibody binding to BDCA2 is 0.5-4, preferably 0.7-1.5, and more preferably 0.8-1.2.

In another preferred embodiment, the number of amino acids added, deleted, modified, and/or substituted is 1-5 (e.g., 1-3, preferably 1-2, more preferably 1).

In another preferred embodiment, the derivative sequence comprises a heavy chain variable region and a light chain variable region, the heavy chain variable region comprises heavy chain complementarity determining regions having the following amino acid sequences:
an H-CDR₁ having an amino acid sequence of SYX1IH, wherein X₁ is I or L; and/or
an H-CDR2 having an amino acid sequence of TIYPGX2GDTSYNQKFKG, wherein X2 is
an amino acid selected from the group consisting of: N, G, A, S, R (preferably G); and/or
the light chain variable region comprising light chain complementarity determining regions having the following amino acid sequences:
   an L-CDR2 having an amino acid sequence of DAETLAX3, wherein X3 is an amino acid selected from the group consisting of: D, I, or E;
   wherein X₁, X₂, and X₃ are not simultaneously I, N, and D, respectively.

In another preferred embodiment, in the derivative sequence, X₁, X₂, and X₃ are selected from the group consisting of:
X₁ is L, X₂ is N, X₃ is I;
X₁ is L, X₂ is N, X₃ is E;
X₁ is L, X₂ is G, X₃ is I;
X₁ is L, X₂ is G, X₃ is E;
X₁ is L, X₂ is R, X₃ is I;
X₁ is L, X₂ is R, X₃ is E;
X₁ is I, X₂ is G, X₃ is I;
X₁ is I, X₂ is G, X₃ is E;
X₁ is I, X₂ is R, X₃ is I; or
X₁ is I, X₂ is R, X₃ is E.

In another preferred embodiment, in the derivative sequence, the heavy chain variable region comprises
an H-CDR1 having an amino acid sequence as shown in SYLIH (SEQ ID NO: 14),
an H-CDR2 having an amino acid sequence as shown in TIYPGGGDTSYNQKFKG (SEQ ID NO: 15),
an H-CDR3 having an amino acid sequence as shown in MGDNEYFDY (SEQ ID NO: 8), and
the light chain variable region comprises
an L-CDR1 having an amino acid sequence as shown in RASGNIHNYLA (SEQ ID NO: 9),
an L-CDR2 having an amino acid sequence as shown in DAETLAE (SEQ ID NO: 20),
an L-CDR3 having an amino acid sequence as shown in QHFWSTPYT (SEQ ID NO: 11).

In another preferred embodiment, in the derivative sequence, the heavy chain variable region comprises
an H-CDR1 having an amino acid sequence as shown in SYIIH (SEQ ID NO: 6),
an H-CDR2 having an amino acid sequence as shown in TIYPGGGDTSYNQKFKG (SEQ ID NO: 15),
an H-CDR3 having an amino acid sequence as shown in MGDNEYFDY (SEQ ID NO: 8), and
the light chain variable region comprises
   an L-CDR1 having an amino acid sequence as shown in RASGNIHNYLA (SEQ ID NO: 9),
   an L-CDR2 having an amino acid sequence as shown in DAETLAE (SEQ ID NO: 20),
   an L-CDR3 having an amino acid sequence as shown in QHFWSTPYT (SEQ ID NO: 11).

In another preferred embodiment, in the derivative sequence, the heavy chain variable region comprises
an H-CDR1 having an amino acid sequence as shown in SYLIH (SEQ ID NO: 14),
an H-CDR2 having an amino acid sequence as shown in TIYPGNGDTSYNQKFKG (SEQ ID NO: 7),
an H-CDR3 having an amino acid sequence as shown in MGDNEYFDY (SEQ ID NO: 8), and
the light chain variable region comprises
   an L-CDR1 having an amino acid sequence as shown in RASGNIHNYLA (SEQ ID NO: 9),
   an L-CDR2 having an amino acid sequence as shown in DAETLAE (SEQ ID NO: 20),
   an L-CDR3 having an amino acid sequence as shown in QHFWSTPYT (SEQ ID NO: 11).

In another preferred embodiment, the antibody is a murine antibody, a chimeric antibody, or a humanized antibody.

In another preferred embodiment, the heavy chain variable region of the antibody further comprises a human-derived framework region, and/or the light chain variable region of the antibody further comprises a human-derived framework region.

In another preferred embodiment, the heavy chain variable region of the antibody further comprises a murine-derived framework region, and/or the light chain variable region of the antibody further comprises a murine-derived framework region.

In another preferred embodiment, the heavy chain constant region is human-derived, and/or the light chain constant region is human-derived.

In another preferred embodiment, the heavy chain constant region is a human antibody heavy chain IgG1 constant region, and the light chain constant region is a human antibody light chain kappa constant region.

In another preferred embodiment, the heavy chain constant region comprises an Fc portion.

In another preferred embodiment, the Fc portion is an Fc variant, and the engineered Fc variant comprises the following mutations: M428L/N434S; G236A/S239D/I332E; S239D/I332E/A330L; S239D/I332E/G236A; S298A; A330L; I332E; E333A; and/or K334A;
wherein the numbering of residues in the Fc variant is according to the EU index of Kabat. In another preferred embodiment, the antibody is selected from the group consisting of: an animal-derived antibody, a chimeric antibody, a humanized antibody, a fully human antibody, or a combination thereof.

In another preferred embodiment, the antibody is a diabody or a single-chain antibody.

In another preferred embodiment, the antibody is a full-length antibody protein or an antigen-binding fragment.

In another preferred embodiment, the antibody is a bispecific antibody or a multispecific antibody.

In another preferred embodiment, the antibody is in the form of a drug conjugate.

In another preferred embodiment, the antibody has an affinity for the antigen BDCA2 of ≤1E-9 M; preferably ≤1E-10 M; more preferably ≤1E-11 M.

In another preferred embodiment, the BDCA2 is human BDCA2; preferably the extracellular domain of human BDCA2.

In another preferred embodiment, the sequence of the extracellular domain of human BDCA2 is as shown in SEQ ID NO: 1.

In another preferred embodiment, the antigen-binding fragment comprises a Fab fragment, an F(ab')2 fragment, an Fv fragment, or an scFv.

In another preferred embodiment, the amino acid sequence of the heavy chain variable region of the antibody or antigen-binding fragment that binds BDCA2 is as shown in SEQ ID NO: 3, and the amino acid sequence of the light chain variable region is as shown in SEQ ID NO: 5.

In another preferred embodiment, the amino acid sequence of the heavy chain variable region of the antibody or antigen-binding fragment that binds BDCA2 is as shown in SEQ ID NO: 12, and the amino acid sequence of the light chain variable region is as shown in SEQ ID NO: 13.

In another preferred embodiment, the amino acid sequence of the heavy chain variable region of the antibody or antigen-binding fragment that binds BDCA2 is selected from SEQ ID NO: 21, 22, 23, 24, or 25, and the amino acid sequence of the light chain variable region is selected from SEQ ID NO: 26 or 27.

In another preferred embodiment, the amino acid sequence of the heavy chain variable region has at least 80%, 85%, 90%, 91%, 92%, 93%, 94%, 95%, 96%, 97%, 98%, or 99% sequence homology or sequence identity with the amino acid sequence as shown in SEQ ID NO: 3, 12, 21, 22, 23, 24, or 25.

In another preferred embodiment, the amino acid sequence of the light chain variable region has at least 80%, 85%, 90%, 91%, 92%, 93%, 94%, 95%, 96%, 97%, 98%, or 99% sequence homology or sequence identity with the amino acid sequence as shown in SEQ ID NO: 5, 13, 26, or 27.

In a second aspect of the present invention, provided is a recombinant protein, wherein the recombinant protein comprises:
(i) the antibody or antigen-binding fragment that binds BDCA2 according to the first aspect of the present invention; and
(ii) optionally, a tag sequence that facilitates expression and/or purification.

In another preferred embodiment, the tag sequence comprises a 6His tag.

In another preferred embodiment, the recombinant protein (or polypeptide) comprises a fusion protein.

In another preferred embodiment, the recombinant protein is a monomer, dimer, or multimer.

In a third aspect of the present invention, provided is a nucleotide molecule, wherein the nucleotide molecule encodes the antibody or antigen-binding fragment that binds BDCA2 according to the first aspect of the present invention.

In another preferred embodiment, the nucleotide sequence encoding the heavy chain variable region is as shown in SEQ ID NO: 2, and the nucleotide sequence encoding the light chain variable region is as shown in SEQ ID NO: 4.

In a fourth aspect of the present invention, provided is an expression vector, wherein the expression vector comprises the nucleotide molecule according to the third aspect of the present invention.

In a fifth aspect of the present invention, provided is a host cell, wherein the host cell comprises the expression vector according to the fourth aspect of the present invention.

In a sixth aspect of the present invention, provided is a method for preparing the antibody or antigen-binding fragment that binds BDCA2 according to the first aspect of the present invention, wherein the method comprises the following steps:
a) culturing the host cell according to the fifth aspect of the present invention under expression conditions to express the antibody or antigen-binding fragment thereof that binds to BDCA2;
b) isolating and purifying the antibody or antigen-binding fragment thereof that binds to BDCA2 described in step a).

In a seventh aspect of the present invention, provided is a composition, wherein the composition comprises the antibody or antigen-binding fragment that binds BDCA2 according to the first aspect of the present invention and a pharmaceutically acceptable carrier.

In an eighth aspect of the present invention, provided is a use of the antibody or antigen-binding fragment that binds BDCA2 according to the first aspect of the present invention or the composition according to the seventh aspect of the present invention in the manufacture of a medicament for treating an inflammatory disease or an autoimmune disease.

In another preferred embodiment, the inflammatory disease or autoimmune disease is selected from the group consisting of: systemic lupus erythematosus, cutaneous lupus erythematosus, discoid lupus, lupus nephritis, cutaneous lupus, rheumatoid arthritis, inflammatory bowel disease, systemic sclerosis (scleroderma), psoriasis, type I diabetes, dermatomyositis, and polymyositis.

In a ninth aspect of the present invention, provided is a CAR construct, wherein the scFv segment of the monoclonal antibody antigen-binding region of the CAR construct is a binding region that specifically binds to BDCA2, and the heavy chain variable region of the scFv comprises:
heavy chain complementarity determining regions H-CDR1, H-CDR2, and H-CDR3, wherein the amino acid sequence of H-CDR1 is as shown in SYIIH (SEQ ID NO: 6), the amino acid sequence of H-CDR2 is as shown in TIYPGNGDTSYNQKFKG (SEQ ID NO: 7), the amino acid sequence of H-CDR3 is as shown in MGDNEYFDY (SEQ ID NO: 8), and
the light chain variable region of the scFv comprises:
   light chain complementarity determining regions L-CDR1, L-CDR2, and L-CDR3, wherein the amino acid sequence of the L-CDR1 is as shown in RASGNIHNYLA (SEQ ID NO: 9), the amino acid sequence of L-CDR2 is as shown in DAETLAD (SEQ ID NO: 10), and the amino acid sequence of L-CDR3 is as shown in QHFWSTPYT (SEQ ID NO: 11); wherein any one of the aforementioned amino acid sequences further optionally comprises a derivative sequence that has undergone addition, deletion, modification, and/or substitution of at least one amino acid and retains BDCA2 binding affinity.

In another preferred embodiment, the derivative sequence comprises a heavy chain variable region and a light chain variable region, the heavy chain variable region comprises heavy chain complementarity determining regions having the following amino acid sequences:
H-CDR1 having an amino acid sequence as shown in SYX₁IH, wherein X₁ is I or L; and/or H-CDR2 having an amino acid sequence as shown in TIYPGX2GDTSYNQKFKG,
wherein X₂ is an amino acid selected from the group consisting of: N, G, A, S, R (preferably G); and/or
the light chain variable region comprising light chain complementarity determining regions having the following amino acid sequences:
   L-CDR2 having an amino acid sequence as shown in DAETLAX₃, wherein X₃ is an amino acid selected from the group consisting of: D, I, or E;
   wherein X₁, X₂, and X₃ are not simultaneously I, N, and D, respectively.

In a tenth aspect of the present invention, provided is a recombinant immune cell, wherein the immune cell expresses an exogenous CAR construct according to the ninth aspect of the present invention.

In an eleventh aspect of the present invention, provided is an antibody-drug conjugate, wherein the antibody-drug conjugate comprises:
(a) an antibody moiety, the antibody moiety comprising the antibody or antigen-binding fragment thereof according to the first aspect of the present invention; and
(b) a conjugate moiety conjugated to the antibody moiety, the conjugate moiety is selected from the group consisting of: a detectable label, a drug, a toxin, a cytokine, a radionuclide, an enzyme, or a combination thereof.

In a twelfth aspect of the present invention, provided is a method for detecting BDCA2 protein in a sample in vitro, wherein the method comprises the steps of:
(1) contacting the sample in vitro with the antibody or antigen-binding fragment thereof according to the first aspect of the present invention or the antibody-drug conjugate according to the eleventh aspect of the present invention;
(2) detecting whether an antigen-antibody complex is formed, wherein formation of a complex indicates the presence of BDCA2 protein in the sample.

In a thirteenth aspect of the present invention, provided is a method for preventing and/or treating a BDCA2-associated disease, the method comprising: administering to a subject in need thereof the BDCA2-binding antibody or antigen-binding fragment thereof according to the first aspect of the present invention, the composition according to the seventh aspect of the present invention, the recombinant immune cell according to the tenth aspect of the present invention, or the antibody-drug conjugate according to the eleventh aspect of the present invention, or a combination thereof.

In another preferred embodiment, the BDCA2-associated disease is selected from inflammatory diseases or autoimmune diseases, including but not limited to systemic lupus erythematosus, cutaneous lupus erythematosus, discoid lupus, lupus nephritis, cutaneous lupus, rheumatoid arthritis, inflammatory bowel disease, systemic sclerosis (scleroderma), psoriasis, type I diabetes, dermatomyositis and polymyositis, or combinations thereof.

It should be understood that each of the above technical features of the present invention and each of the technical features specifically described below (e.g., in the Examples) can be combined with each other within the scope of the present invention to thus form new or preferred technical solutions, which will not be repeated here one by one due to space limitation.

### BRIEF DESCRIPTION OF DRAWINGS

Figure 1A to Figure 1B show the binding capability of murine antibodies to human BDCA2-Fc protein.
Figure 2A to Figure 2B show the determination of binding affinity of murine antibodies to the stable transfected cell line 293FT-BDCA2.
Figure 3 shows the binding capability of human-mouse chimeric antibodies to human BDCA2-his protein.
Figure 4 shows the inhibitory effect of human-mouse chimeric antibodies on CpG-A-induced IFNα secretion by PBMCs.
Figure 5 shows the effect of different mutation sites on the affinity of antibody 230 as screened by ELISA.
Figure 6 shows the affinity of anti-BDCA2 antibody mutants to BDCA2-his antigen protein as detected by ELISA.
Figure 7 shows the inhibitory effect of candidate antibodies on CpG-A-induced IFNα secretion by PBMCs using PBMCs.
Figure 8 shows the inhibitory effect of candidate antibodies on CpG-A-induced IgM secretion by PBMCs.
Figure 9 shows the inhibitory effect of candidate antibodies on IFNα secretion in mice in vivo.

### DETAILED DESCRIPTION OF THE PREFERRED EMBODIMENTS

Through extensive and in-depth research and extensive screening, the inventors of the present invention have unexpectedly discovered a series of murine antibodies that exhibit excellent binding activity to the target antigen BDCA2-Fc. Further candidate antibodies were selected, and through humanization and CDR modification of the antibodies, the obtained humanized antibodies exhibited affinity and expression levels comparable to or better than those of the parental antibodies. The antibodies of the present invention can effectively inhibit CpG-A-induced secretion of IFNα and IgM by PBMCs, and their activity is significantly superior to that of the positive control antibody Litifilimab. The present invention has been completed on this basis.

### Terms

Unless otherwise defined, all technical and scientific terms used herein have the same meaning as commonly understood by those of ordinary skill in the art to which the present invention belongs.

The term "about" may refer to a value or set that is within an acceptable error range for a particular value or set as determined by those of ordinary skill in the art, and the value or set partially depends on how the value or set is measured or determined.

As used herein, the terms "contain" or "comprise (comprising)" may be open-ended, semi-closed and closed-ended. In other words, the terms also include "consisting essentially of" or "consisting of."

### Antibody

As used herein, the term "antibody" or "immunoglobulin" refers to a heterotetrameric glycoprotein of approximately 150,000 Daltons with the same structural characteristics, composed of two identical light chains (L) and two identical heavy chains (H). Each light chain is linked to a heavy chain by a covalent disulfide bond, while the number of disulfide bonds between heavy chains varies among different immunoglobulin isotypes. Each heavy chain and light chain also has regularly spaced intrachain disulfide bonds. One end of each heavy chain has a variable region (VH), followed by multiple constant regions. One end of each light chain has a variable region (VL), and the other end has a constant region; the constant region of the light chain aligns with the first constant region of the heavy chain, and the variable region of the light chain aligns with the variable region of the heavy chain. Specific amino acid residues form an interface between the variable regions of the light and heavy chains.

As used herein, the term "variable" denotes that some moieties of the variable region of an antibody are somewhat different in sequence, which contribute to the binding and specificity of various particular antibodies to their particular antigens. However, variability is not uniformly distributed across the variable region of an antibody. It is concentrated in three fragments in the so-called complementary determining regions (CDRs) or hypervariable regions in the light and heavy chain variable regions. A conserved moiety in the variable region is called the framework region (FR). The variable regions of the natural heavy and light chains each comprise four FRs, which are roughly in a β-sheet configuration and linked by three CDRs that form a linking loop, and which in some cases may form a partially β-sheet structure. The CDRs in each chain are close together through the FRs and form, together with the CDRs of the other chain, the antigen-binding site of the antibody (see Kabat et al., NIH Publ. No. 91-3242, Vol. I, pp. 647-669 (1991)). The constant regions are not directly involved in antibody-antigen binding, but exhibit different effector functions. For example, they are involved the antibody-dependent cytotoxicity of the antibody.

The "light chains" of vertebrate antibodies (immunoglobulins) can be assigned to one of two distinct types, called kappa (κ) and lambda (λ), based on the amino acid sequences of their constant regions. Immunoglobulins can be classified into different classes based on the amino acid sequences of their heavy chain constant regions. There are five major classes of immunoglobulins: IgA, IgD, IgE, IgG, and IgM, and some of these can be further divided into subclasses (isotypes), such as IgG1, IgG2, IgG3, IgG4, IgA1, and IgA2. The heavy chain constant regions corresponding to the different classes of immunoglobulins are designated α, δ, ε, γ, and µ, respectively. The subunit structures and three-dimensional configurations of different classes of immunoglobulins are well known to those skilled in the art.

Generally, the antigen-binding characteristics of an antibody can be described by three specific regions located in the variable regions of the heavy and light chains, referred to as complementarity-determining regions (CDRs), which are interspersed with four framework regions (FRs); the amino acid sequences of the four FRs are relatively conserved and do not directly participate in binding reactions. These CDRs form loop structures that are brought into close spatial proximity by the β-sheets formed by the intervening FRs, and the CDRs on the heavy chain and the corresponding CDRs on the light chain constitute the antigen-binding site of the antibody. The amino acids constituting the FR or CDR regions can be determined by comparing the amino acid sequences of antibodies of the same type.

The present invention encompasses not only intact antibodies but also fragments of antibodies having immunological activity or fusion proteins formed by antibodies and other sequences. Accordingly, the present invention also includes fragments, derivatives, and analogs of the antibodies.

In the present invention, antibodies include murine, chimeric, humanized, or fully human antibodies prepared using techniques well known to those skilled in the art. Recombinant antibodies, such as chimeric and humanized monoclonal antibodies, comprising both human and non-human portions, can be obtained by standard DNA recombination techniques, and all such antibodies are useful. A chimeric antibody is a molecule in which different portions are derived from different animal species, for example, a chimeric antibody having a variable region derived from a murine monoclonal antibody and a constant region derived from a human immunoglobulin (see, for example, U.S. Patent No. 4,816,567 and U.S. Patent No. 4,816,397, which are incorporated herein by reference in their entirety). A humanized antibody refers to an antibody molecule derived from a non-human species, comprising one or more complementarity determining regions (CDRs) derived from a non-human species and framework regions derived from a human immunoglobulin molecule (see U.S. Patent No. 5,585,089, which is incorporated herein by reference in its entirety). These chimeric and humanized monoclonal antibodies can be prepared using DNA recombination techniques well known in the art.

In the present invention, the antibody may be monospecific, bispecific, trispecific, or of higher multispecificity.

In the present invention, the antibodies of the present invention also include conservative variants thereof, which refer to polypeptides formed by substitution of up to 10, preferably up to 8, preferably up to 5, and optimally up to 3 amino acids with amino acids having similar or analogous properties, as compared to the amino acid sequence of the bispecific antibody of the present invention. These conserved variant polypeptides are preferably produced by amino acid substitution according to Table A.

**Table A**

| Initial residues | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

### Anti-BDCA2 Antibody

In the present invention, the antibody is an anti-BDCA2 antibody. The present invention provides an antibody with high specificity and high affinity against BDCA2, comprising a heavy chain and a light chain, wherein the heavy chain comprises a heavy chain variable region (VH) amino acid sequence, and the light chain comprises a light chain variable region (VL) amino acid sequence.

Wherein, the heavy chain variable region comprises a heavy chain complementarity determining region H-CDR1, H-CDR2, and H-CDR3,

The amino acid sequence of H-CDR1 is as shown in SYX₁IH (SEQ ID NO: 30),

The amino acid sequence of H-CDR2 is as shown in TIYPGX₂GDTSYNQKFKG (SEQ ID NO: 31),
the amino acid sequence of H-CDR3 is as shown in MGDNEYFDY (SEQ ID NO: 8), and the light chain variable region comprises light chain complementarity determining regions L-CDR1, L-CDR2, and L-CDR3; wherein,
the amino acid sequence of L-CDR1 is as shown in RASGNIHNYLA (SEQ ID NO: 9),
the amino acid sequence of L-CDR2 is as shown in DAETLAX₃ (SEQ ID NO: 32),
the amino acid sequence of L-CDR3 is as shown in QHFWSTPYT (SEQ ID NO: 11);
wherein, X₁ is I or L; X₂ is an amino acid selected from the group consisting of: N, G, A, S,
R; and/or X₃ is an amino acid selected from the group consisting of: D, I, or E.

Preferably,
the amino acid sequence of H-CDR1 is as shown in SYIIH (SEQ ID NO: 6),
the amino acid sequence of H-CDR2 is as shown in TIYPGNGDTSYNQKFKG (SEQ ID NO: 7),
the amino acid sequence of H-CDR3 is as shown in MGDNEYFDY (SEQ ID NO: 8), and
the light chain variable region comprises light chain complementarity determining regions L-CDR1, L-CDR2, and L-CDR3; wherein,
the amino acid sequence of L-CDR1 is as shown in RASGNIHNYLA (SEQ ID NO: 9),
the amino acid sequence of L-CDR2 is as shown in DAETLAD (SEQ ID NO: 10),
the amino acid sequence of L-CDR3 is as shown in QHFWSTPYT (SEQ ID NO: 11);
wherein any one of the aforementioned amino acid sequences further optionally comprises a derivative sequence that optionally undergoes addition, deletion, modification, and/or substitution of at least one amino acid and is capable of retaining BDCA2 binding affinity.

In another preferred embodiment, the sequence formed by addition, deletion, modification, and/or substitution of at least one amino acid is preferably an amino acid sequence with homology or sequence identity of at least 80%, more preferably at least 85%, even more preferably at least 90%, and most preferably at least 95%.

Methods for determining sequence homology or identity well-known to those of ordinary skill in the art include, but are not limited to: Computational Molecular Biology, Lesk, A.M. ed., Oxford University Press, New York, 1988; Biocomputing: Informatics and Genome Projects, Smith, D.W. ed., Academic Press, New York, 1993; Computer Analysis of Sequence Data, Part I, Griffin, A.M. and Griffin, H.G. eds., Humana Press, New Jersey, 1994*;* Sequence Analysis in Molecular Biology, von Heinje, G., Academic Press, 1987; and Sequence Analysis Primer, Gribskov, M. and Devereux, J. eds., M Stockton Press, New York, 1991, and Carillo, H. and Lipman, D., SIAM J. Applied Math., 48:1073 (1988). Preferred methods for determining identity are designed to give the largest match between the sequences being tested. Methods for determining identity are compiled in publicly available computer programs. Preferred computer program methods for determining identity between two sequences include, but are not limited to: the GCG program package (Devereux, J. et al., 1984), BLASTP, BLASTN, and FASTA (Altschul, S.F. et al., 1990). The BLASTX program is publicly available from NCBI and other sources (BLAST Manual, Altschul, S. et al., NCBI NLM NIH Bethesda, Md. 20894; Altschul, S. et al., 1990). The well-known Smith Waterman algorithm may also be used to determine identity.

Preferably, the antibody described herein is one or more of a full-length antibody protein, an antigen-antibody binding domain protein fragment, a bispecific antibody, a multispecific antibody, a single-chain antibody fragment (scFv), a single-domain antibody (sdAb), and a single-domain antibody (Signle-domain antibody), as well as monoclonal antibodies or polyclonal antibodies prepared from the aforementioned antibodies. The monoclonal antibody may be developed through various approaches and techniques, including hybridoma technology, phage display technology, single lymphocyte gene cloning technology, etc., with the mainstream method being the preparation of monoclonal antibodies from wild-type or transgenic mice via hybridoma technology.

The full-length antibody protein is a conventional full-length antibody protein in the art, comprising a heavy chain variable region, a light chain variable region, a heavy chain constant region, and a light chain constant region. The heavy chain variable region and light chain variable region of the protein, together with a human-derived heavy chain constant region and a human-derived light chain constant region, constitute a full-length fully human antibody protein. Preferably, the full-length antibody protein is IgG1, IgG2, IgG3, or IgG4.

The antibody (anti-BDCA2 antibody) in the present invention may be a full-length protein (such as IgG1, IgG2a, IgG2b, or IgG2c) or a protein fragment containing an antigen-antibody binding domain (e.g., Fab, F(ab'), sdAb, ScFv fragment).

The antibody (anti-BDCA2 antibody) in the present invention may be a wild-type protein or a mutant protein engineered through specific mutations to achieve a particular effect, such as eliminating the effector function of the antibody via mutation.

The antibody of the present invention may be a double-chain or single-chain antibody and may be selected from animal-derived antibodies, chimeric antibodies, humanized antibodies, more preferably humanized antibodies, human-animal chimeric antibodies, and even more preferably fully humanized antibodies.

The antibody derivative of the present invention may be a single-chain antibody and/or an antibody fragment, such as: Fab, Fab', (Fab')2, or other known antibody derivatives in the art, as well as any one or more of IgA, IgD, IgE, IgG, and IgM antibodies or antibodies of other subtypes.

The single-chain antibody is a conventional single-chain antibody in the art, comprising a heavy chain variable region, a light chain variable region, and a short peptide of 15-20 amino acids.

Among these, the animal is preferably a mammal, such as a mouse.

As used herein, "Fc variant" or "variant Fc" refers to a protein comprising modifications in the Fc domain. Fc variants of the present invention are defined by the Aminoacid modifications constituting them. Therefore. For example, N434S or 434S refers to an Fc variant having a substitution at position 434 relative to the parent Fc polypeptide, Serinewherein the numbering is according to the EU index. Similarly, M428L/N434S defines an Fc variant having substitutions M428L and N434S. Relative to the parent Fc polypeptide. The identity of the wild-type Aminoacid may be non-specific, in which case the above variant is referred to as 428L/434S.Note that the order of substitutions is arbitrarily provided, that is, for example, 428L/434S is the same Fc variant as M428L/N434S, and so forth. For all positions discussed in the present invention, the numbering is according to the EU index. The EU index or EU index as in Kabat or EU numbering scheme refers to the numbering of the EU antibody (Edelman et al., 1969, Proc Natl Acad Sci USA 63:78-85, which is incorporated herein by reference in its entirety). The modification may be an addition, deletion, or substitution. Substitutions may include naturally occurringAminoacids and non-naturally occurringaminoacids. Variants may comprise non-natural amino acids. Examples include US6,586,207; WO98/48032; WO03/073238; US2004-0214988A1; WO05/35727A2; WO05/74524A2; J.W. Chin et al., (2002), Journal of the American Chemical Society 124:9026-9027; J.W. Chin and P.G. Schultz, (2002), ChemBioChem 11:1135-1137; J.W. Chin et al., (2002), PNAS United States of America 99:11020-11024; and L. Wang and P.G. Schultz, (2002), Chem. 1-10, all of which are incorporated herein by reference in their entirety.

The antibody of the present invention may be a chimeric antibody, humanized antibody, CDR-grafted and/or modified antibody targeting BDCA2 (e.g., human BDCA2).

Furthermore, the CDR regions of the murine antibody are grafted onto selected humanized templates, replacing the CDR regions of the human template. A chimeric antibody is obtained by recombining the heavy chain variable region with a human IgG1 constant region and the light chain variable region with a human kappa chain constant region. Based on the three-dimensional structure of the antibody, back mutations were performed on buried residues, residues with direct interactions with the CDR regions, and residues significantly influencing the conformation of the antibody's VL and VH, resulting in the generation of multiple humanized antibodies.

In the above content of the present invention, the number of amino acids added, deleted, modified, and/or substituted is preferably no more than 40% of the total number of amino acids in the initial amino acid sequence, more preferably no more than 35%, even more preferably 1-33%, even more preferably 5-30%, even more preferably 10-25%, and even more preferably 15-20%.

In the above content of the present invention, more preferably, the number of amino acids added, deleted, modified, and/or substituted may be 1-7, more preferably 1-5, more preferably 1-3, and more preferably 1-2.

Preferably, the humanized antibody comprises a heavy chain variable region and a light chain variable region, wherein, relative to the murine antibody, the heavy chain variable region comprises a heavy chain complementarity determining region having the following amino acid sequence:
H-CDR1 having an amino acid sequence as shown in SYX₁IH, wherein X₁ is I or L; and/or
H-CDR2 having an amino acid sequence as shown in TIYPGX2GDTSYNQKFKG,
wherein X₂ is an amino acid selected from the group consisting of: N, G, A, S, R (preferably G); and/or
the light chain variable region comprising light chain complementarity determining regions having the following amino acid sequences:
   L-CDR2 having an amino acid sequence as shown in DAETLAX₃, wherein X₃ is an amino acid selected from the group consisting of: D, I, or E;
   wherein X₁, X₂, and X₃ are not simultaneously I, N, and D, respectively.

In another preferred embodiment, the heavy chain variable region and light chain variable region of the humanized antibody comprise heavy chain complementarity determining regions and light chain complementarity determining regions selected from the group consisting of:
(1) H-CDR1 selected from the group consisting of:
   SYIIH (SEQ ID NO: 6)
   SYLIH (SEQ ID NO: 14)
(2) H-CDR2 selected from the group consisting of:
   TIYPGNGDTSYNQKFKG (SEQ ID NO: 7)
   TIYPGGGDTSYNQKFKG (SEQ ID NO: 15)
   TIYPGAGDTSYNQKFKG (SEQ ID NO: 16)
   TIYPGSGDTSYNQKFKG (SEQ ID NO: 17)
   TIYPGRGDTSYNQKFKG (SEQ ID NO: 18)
(3) H-CDR3 is MGDNEYFDY (SEQ ID NO: 8)
(4) L-CDR1 is RASGNIHNYLA (SEQ ID NO: 9)
(5) L-CDR2 selected from the group consisting of:
   DAETLAD (SEQ ID NO: 10)
   DAETLAI (SEQ ID NO: 19)
   DAETLAE (SEQ ID NO: 20)
(6) L-CDR3 is QHFWSTPYT (SEQ ID NO: 11)

In another preferred embodiment, the amino acid sequence of the heavy chain variable region comprises an amino acid sequence as shown in SEQ ID NO: 3, 12, 21, 22, 23, 24, or 25, or has 80%, 85%, 90%, 91%, 92%, 93%, 94%, 95%, 96%, 97%, 98%, or 99% sequence homology or sequence identity thereto.

In another preferred embodiment, the amino acid sequence of the light chain variable region comprises an amino acid sequence as shown in SEQ ID NO: 5, 13, 26, or 27, or has at least 80%, 85%, 90%, 91%, 92%, 93%, 94%, 95%, 96%, 97%, 98%, or 99% sequence homology or sequence identity thereto.

In another preferred embodiment, the amino acid sequence of the heavy chain variable region (VH) and/or the light chain variable region (VL) of the anti-BDCA2 antibody is as shown in Table B below:

**Table B**

| Antibody Name | VH Sequence ID | VL Sequence ID |
|---|---|---|
| 23C10A6B5 | 3 | 5 |
| Humanized Antibody 230 | 12 | 13 |
| 230IL-NG-DE | 22 | 27 |
| 230NG-DE | 24 | 27 |
| 230IL-DE | 21 | 27 |
| 230IL-DI | 21 | 26 |
| 230IL-NG-DI | 22 | 26 |
| 230IL-NR-DI | 23 | 26 |
| 230IL-NR-DE | 23 | 27 |
| 230NG-DI | 24 | 26 |
| 230NR-DI | 25 | 26 |
| 230NR-DE | 25 | 27 |

### Recombinant Protein

The present invention further provides a recombinant protein comprising the heavy chain CDR1 (H-CDR1), heavy chain CDR2 (H-CDR2), and heavy chain CDR3 (H-CDR3) of the anti-BDCA2 antibody according to the first aspect of the present invention, and/or the light chain CDR1 (L-CDR1), light chain CDR2 (L-CDR2), and light chain CDR3 (L-CDR3) of the anti-BDCA2 antibody.

The recombinant protein further comprises an antibody heavy chain constant region and/or an antibody light chain constant region, wherein the antibody heavy chain constant region is conventional in the art, preferably a rat-derived antibody heavy chain constant region or a human-derived antibody heavy chain constant region, and more preferably a human-derived antibody heavy chain constant region. The antibody light chain constant region is conventional in the art, preferably a rat-derived antibody light chain constant region or a human-derived antibody light chain constant region, and more preferably a human-derived antibody light chain constant region.

The recombinant protein is a conventional protein in the art, preferably one or more of a full-length antibody protein, an antigen-antibody binding domain protein fragment, a bispecific antibody, a multispecific antibody, a single-chain antibody fragment (scFv), a single-domain antibody (sdAb), and a single-domain antibody (Signle-domain antibody), as well as monoclonal antibodies or polyclonal antibodies prepared from the aforementioned antibodies. The monoclonal antibody may be developed through various approaches and techniques, including hybridoma technology, phage display technology, single lymphocyte gene cloning technology, etc., with the mainstream method being the preparation of monoclonal antibodies from wild-type or transgenic mice via hybridoma technology.

The full-length antibody protein is a conventional full-length antibody protein in the art, comprising a heavy chain variable region, a light chain variable region, a heavy chain constant region, and a light chain constant region. The heavy chain variable region and light chain variable region of the protein, together with a human-derived heavy chain constant region and a human-derived light chain constant region, constitute a full-length fully human antibody protein. Preferably, the full-length antibody protein is IgG1, IgG2, IgG3, or IgG4.

The single-chain antibody is a conventional single-chain antibody in the art, comprising a heavy chain variable region, a light chain variable region, and a short peptide of 15-20 amino acids.

The antigen-antibody binding domain protein fragment is a conventional antigen-antibody binding domain protein fragment in the art, comprising a light chain variable region, a light chain constant region, and the Fd segment of the heavy chain constant region.

Preferably, the antigen-antibody binding domain protein fragment is Fab or F(ab').

The single-domain antibody is a conventional single-domain antibody in the art, comprising a heavy chain variable region and a heavy chain constant region.

The single-region antibody is a conventional single-region antibody in the art, comprising only a heavy chain variable region.

The method for preparing the recombinant protein is a conventional preparation method in the art. Preferably, the preparation method comprises: isolating the protein from an expression transformant that recombinantly expresses the protein, or obtaining the protein by artificial synthesis of the protein sequence. The isolation from an expression transformant that recombinantly expresses the protein preferably comprises the following method: cloning a nucleic acid molecule encoding the protein and carrying a point mutation into a recombinant vector, transforming the resulting recombinant vector into a transformant to obtain a recombinant expression transformant, and isolating and purifying the recombinant protein by culturing the resulting recombinant expression transformant.

### Nucleic Acid

The present invention further provides a nucleic acid encoding the aforementioned antibody (e.g., an anti-BDCA2 antibody), recombinant protein, or the heavy chain variable region or light chain variable region of the anti-BDCA2 antibody.

The method for preparing the nucleic acid is a conventional preparation method in the art, preferably comprising the following steps: obtaining a nucleic acid molecule encoding the aforementioned protein by gene cloning technology, or obtaining a nucleic acid molecule encoding the aforementioned protein by artificial full-sequence synthesis.

Those skilled in the art understand that the base sequence encoding the amino acid sequence of the aforementioned protein may be appropriately substituted, deleted, altered, inserted, or added to provide a homolog of the polynucleotide. In the present invention, a homolog of the polynucleotide can be prepared by substituting, deleting, or adding one or more bases of the gene encoding the protein sequence within a range that preserves antibody activity.

### Vector

The present invention further provides a recombinant expression vector comprising the nucleic acid.

The recombinant expression vector can be obtained by conventional methods in the art, namely: by ligating the nucleic acid molecule of the present invention to various expression vectors for construction. The expression vector is any conventional vector in the art, provided that it is capable of accommodating the aforementioned nucleic acid molecule. Preferably, the vector includes various plasmids, cosmids, phagemids, or viral vectors.

The present invention further provides a recombinant expression transformant comprising the aforementioned recombinant expression vector.

The method for preparing the recombinant expression transformant is a conventional preparation method in the art, preferably comprising: transforming the aforementioned recombinant expression vector into a host cell to obtain the transformant. The host cell is any conventional host cell in the art, as long as it allows stable autonomous replication of the aforementioned recombinant expression vector and enables effective expression of the carried nucleic acid. Preferably, the host cell is E. coli TG1 or E. coli BL21 (for expressing single-chain antibodies or Fab antibodies), or HEK293 or CHO cells (for expressing full-length IgG antibodies). The preferred recombinant expression transformant of the present invention can be obtained by transforming the aforementioned recombinant expression plasmid into a host cell. The transformation method is a conventional transformation method in the art, preferably chemical transformation, heat shock, or electroporation.

### Preparation of Antibodies

The sequence of the DNA molecule encoding the antibody or fragment thereof of the present invention can be obtained by conventional techniques, such as PCR amplification or genomic library screening. Furthermore, the coding sequences of the light chain and heavy chain can be fused together to form a single-chain antibody.

Once the related sequence has been obtained, the recombination method may be used to obtain the sequence on a large scale. This is typically done by cloning into a vector, transferring into a cell, and then isolating the related sequence from proliferated host cells by a conventional method.

Additionally, the relevant sequences can be synthesized artificially, especially when the fragment length is short. Typically, a fragment with a very long sequence can be obtained by first synthesizing a plurality of small fragments and then ligating them.

At present, it is already possible to obtain a DNA sequence encoding the antibody of the present invention (or a fragment thereof, or a derivative thereof) exclusively by chemical synthesis. Then, this DNA sequence can be introduced into various existing DNA molecules (or, for example, vectors) and cells known in the art. In addition, mutations may be introduced into the protein sequence of the present invention by chemical synthesis.

The present invention further relates to vectors comprising appropriate DNA sequences as described above, as well as appropriate promoters or control sequences. These vectors can be used to transform appropriate host cells to enable them to express the protein.

The host cells may be prokaryotic cells, such as bacterial cells; or lower eukaryotic cells, such as yeast cells; or higher eukaryotic cells, such as mammalian cells. Preferred animal cells include, but are not limited to: CHO-S and HEK-293 cells.

Typically, the transformed host cells are cultured under conditions suitable for the expression of the antibody of the present invention. The antibody of the present invention is then purified using conventional immunoglobulin purification steps, such as protein A-Sepharose chromatography, hydroxyapatite chromatography, gel electrophoresis, dialysis, ion-exchange chromatography, hydrophobic interaction chromatography, size-exclusion chromatography, or affinity chromatography, and other conventional separation and purification methods well-known to those skilled in the art.

The resulting monoclonal antibodies can be characterized using conventional methods. For example, the binding specificity of the monoclonal antibodies can be determined by immunoprecipitation or in vitro binding assays, such as radioimmunoassay (RIA) or enzyme-linked immunosorbent assay (ELISA). The binding affinity of the monoclonal antibodies can be determined, for example, by Scatchard analysis as described in Munson et al., Anal. Biochem., 107:220 (1980).

The antibodies of the present invention may be expressed intracellularly, on the cell membrane, or secreted extracellularly. The recombinant proteins may be isolated and purified by various separation methods using their physical, chemical and other properties, if desired. These methods are well known to those skilled in the art. Examples of these methods include but are not limited to: conventional renaturation, treatment with a protein precipitant (a salting-out method), centrifugation, osmotic lysis, ultrasonication, ultra-centrifugation, molecular sieve chromatography (gel filtration), adsorption chromatography, ion exchange chromatography, high-performance liquid chromatography (HPLC), and various other liquid chromatography techniques, and combinations of these methods.

### Antibody-Drug Conjugate (ADC)

The present invention also provides an antibody-drug conjugate (ADC) based on the antibody of the present invention.

Typically, the antibody-drug conjugate comprises the antibody and an effector molecule, wherein the antibody is conjugated to the effector molecule, preferably by chemical conjugation. The effector molecule is preferably a therapeutically active drug. Furthermore, the effector molecule may be one or more selected from the group consisting of toxic proteins, chemotherapeutic agents, small molecule drugs, and radionuclides.

The conjugation between the antibody of the present invention and the effector molecule may be achieved via a coupling agent. Examples of the coupling agent may include any one or more selected from the group consisting of non-selective coupling agents, carboxyl group-based coupling agents, peptide linkers, and disulfide bond-based coupling agents. The non-selective coupling agent refers to a compound that forms a covalent bond between the effector molecule and the antibody, such as glutaraldehyde. The carboxyl group-based coupling agent may be any one or more selected from the group consisting of cis-aconitic anhydride-based coupling agents (e.g., cis-aconitic anhydride) and acyl hydrazone-based coupling agents (with the conjugation site being an acyl hydrazone). Certain residues on the antibody (such as Cys or Lys) are used for conjugation with various functional groups, including imaging agents (e.g., chromophores and fluorophores), diagnostic agents (e.g., MRI contrast agents and radioisotopes), stabilizers (e.g., glycol polymers), and therapeutic agents. The antibody may be conjugated to a functional agent to form an antibody-functional agent conjugate. A functional agent (e.g., a drug, detection reagent, or stabilizer) is conjugated (covalently linked) to the antibody. The functional agent may be linked to the antibody directly or indirectly via a linker.

The antibody may be conjugated to a drug to form an antibody-drug conjugate (ADC). Typically, an ADC comprises a linker between the drug and the antibody. The linker may be a cleavable or non-cleavable linker. A cleavable linker is typically susceptible to degradation under intracellular conditions, for example, where the linker is cleaved at the target site to release the drug from the antibody. Suitable cleavable linkers include, for example, enzymatically cleavable linkers, which include peptide-based linkers that can be cleaved by intracellular proteases (e.g., lysosomal or endosomal proteases), or carbohydrate linkers such as glucuronide-containing linkers that can be cleaved by glucuronidase. Peptide-based linkers may include, for example, dipeptides such as valine-citrulline, phenylalanine-lysine, or valine-alanine. Other suitable cleavable linkers include, for example, pH-sensitive linkers (e.g., linkers that hydrolyze at pH less than 5.5, such as hydrazone linkers) and linkers that degrade under reducing conditions (e.g., disulfide linkers). Non-cleavable linkers typically release the drug under conditions where the antibody is proteolytically degraded.

Prior to conjugation to the antibody, the linker possesses reactive functional groups capable of reacting with certain amino acid residues, and conjugation is achieved through these reactive functional groups. Sulfhydryl-specific reactive functional groups are preferred and include, for example: maleimide compounds; haloacetamides (e.g., iodo-, bromo-, or chloro-); haloesters (e.g., iodo-, bromo-, or chloro-); halomethyl ketones (e.g., iodo-, bromo-, or chloro-); benzyl halides (e.g., iodo-, bromo-, or chloro-); vinyl sulfones; pyridyl disulfides; mercury derivatives such as 3,6-bis(mercurymethyl)dioxane with counterions being acetate, chloride, or nitrate; and polymethylene dimethyl sulfide thiosulfonates. The linker may include, for example, a maleimide conjugated to the antibody via a thiosuccinimide linkage.

The drug may be any cytotoxic, cytostatic, or immunosuppressive agent. In embodiments, the linker conjugates the antibody and the drug, and the drug possesses a functional group capable of bonding with the linker. For example, the drug may possess an amino, carboxyl, sulfhydryl, hydroxyl, or ketone group capable of bonding with the linker. In cases where the drug is directly conjugated to the linker, the drug possesses a reactive functional group prior to conjugation to the antibody.

Useful classes of drugs include, for example, antitubulin agents, DNA minor groove binders, DNA replication inhibitors, alkylating agents, antibiotics, antifolates, antimetabolites, chemosensitizers, topoisomerase inhibitors, vinca alkaloids, and the like. Examples of particularly useful classes of cytotoxic agents include, for example, DNA minor groove binders, DNA alkylating agents, and tubulin inhibitors; typical cytotoxic agents include, for example, auristatins, camptothecins, Duocarmycins/duocarmycins, etoposides, maytansines and maytansinoids (e.g., DM1 and DM4), taxanes, benzodiazepines or benzodiazepine-containing drugs (e.g., pyrrolo[1,4]benzodiazepines (PBDs), indolinobenzodiazepines and oxazolidinobenzodiazepines), and vinca alkaloids. In the present invention, a drug-linker may be used to form an ADC in a single step. In other embodiments, bifunctional linker compounds may be used to form an ADC in a two-step or multi-step process. For example, a cysteine residue reacts with a reactive moiety of the linker in a first step, and in a subsequent step, a functional group on the linker reacts with the drug to form the ADC.

Typically, the functional group on the linker is selected to facilitate specific reaction with a suitable reactive group on the drug moiety. As a non-limiting example, azide-based moieties may be used to specifically react with reactive alkyne groups on the drug moiety. The drug is covalently attached to the linker via 1,3-dipolar cycloaddition between the azide and the alkyne. Other useful functional groups include, for example: ketones and aldehydes (suitable for reaction with hydrazides and alkoxyamines); phosphines (suitable for reaction with azides); isocyanates and isothiocyanates (suitable for reaction with amines and alcohols); and activated esters, such as N-hydroxysuccinimide esters (suitable for reaction with amines and alcohols). These and other conjugation strategies, such as those described in Bioconjugate Techniques, Second Edition (Elsevier), are well known to those skilled in the art. Those skilled in the art will appreciate that for the selective reaction of the drug moiety and the linker, when a complementary pair of reactive functional groups is selected, each member of the complementary pair can be used either on the linker or on the drug.

The present invention also provides a method for preparing an ADC, which may further comprise: combining the antibody with a drug-linker compound under conditions sufficient to form an antibody conjugate (ADC).

In certain embodiments, the method of the invention comprises: combining the antibody with a bifunctional linker compound under conditions sufficient to form an antibody-linker conjugate. In these embodiments, the method of the invention further comprises: combining the antibody-linker conjugate with a drug moiety under conditions sufficient to covalently attach the drug moiety to the antibody via the linker.

In some embodiments, the antibody-drug conjugate (ADC) is represented by the following formula: 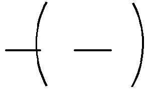 wherein:
Ab is an antibody,
LU is a linker;
D is a drug;
and the subscript p is a value selected from 1 to 8.

### Pharmaceutical Compositions

The present invention also provides a composition. In a preferred embodiment, the composition is a pharmaceutical composition comprising the aforementioned antibody or an active fragment thereof, or a fusion protein thereof, or an ADC thereof, or a corresponding immune cell, and a pharmaceutically acceptable carrier. Typically, these substances may be formulated in a non-toxic, inert, and pharmaceutically acceptable aqueous carrier medium, with the pH typically of about 5-8, and preferably about 6-8, although the pH may vary depending on the nature of the substance to be formulated and the condition to be treated.

The prepared pharmaceutical composition may be administered via conventional routes, including (but not limited to): intratumoral, intraperitoneal, intravenous, or topical administration. Typically, the route of administration of the pharmaceutical composition of the present invention is preferably injection or oral administration. The injection administration preferably includes routes such as intravenous injection, intramuscular injection, intraperitoneal injection, intradermal injection, or subcutaneous injection. The pharmaceutical composition is of various conventional dosage forms in the art, preferably in solid, semi-solid, or liquid form, and may be an aqueous solution, non-aqueous solution, or suspension, more preferably tablets, capsules, granules, injections, or infusions.

The antibody of the present invention may also be expressed intracellularly by a nucleotide sequence for use in cell therapy, for example, the antibody is used in chimeric antigen receptor T cell immunotherapy (CAR-T) or the like.

The pharmaceutical composition of the present invention is a pharmaceutical composition for preventing and/or treating diseases associated with abnormal BDCA2 expression or function.

The pharmaceutical composition of the present invention can be directly used to bind BDCA2 protein molecules, and thus can be used for preventing and treating diseases such as tumors.

The pharmaceutical composition of the present invention comprises a safe and effective amount (e.g., 0.001-99 wt%, preferably 0.01-90 wt%, more preferably 0.1-80 wt%) of the aforementioned monoclonal antibody (or a conjugate thereof) of the present invention, and a pharmaceutically acceptable carrier or excipient. Such carriers include (but are not limited to): saline, buffer solutions, glucose, water, glycerol, ethanol, and their combination. The pharmaceutical preparation should match the route of administration. The pharmaceutical composition of the present invention may be prepared into an injectable form, for example, by conventional methods using normal saline or aqueous solutions containing glucose and other excipients. The pharmaceutical composition in a form such as an injection or a solution should be manufactured under sterile conditions. The dosage of an active ingredient is a therapeutically effective amount, for example, about 1 µg/kg body weight to about 5 mg/kg body weight per day. In addition, the polypeptide of the present invention may also be used in conjunction with other therapeutic agents.

In the present invention, preferably, the pharmaceutical composition of the present invention further comprises one or more pharmaceutically acceptable carriers. The pharmaceutically acceptable carrier is a conventional pharmaceutically acceptable carrier in the art, and the pharmaceutically acceptable carrier may be any suitable physiologically or pharmaceutically acceptable pharmaceutical excipient. The pharmaceutical excipient is a conventional pharmaceutical excipient in the art, preferably including pharmaceutically acceptable excipients, fillers, or diluents. More preferably, the pharmaceutical composition comprises 0.01-99.99% of the aforementioned protein and 0.01-99.99% of a pharmaceutically acceptable carrier, wherein the percentages are mass percentages relative to the pharmaceutical composition.

In the present invention, preferably, the administration amount of the pharmaceutical composition is an effective amount, which is an amount capable of alleviating or delaying the progression of a disease, degenerative condition, or injury-related disorder. The effective amount may be determined on an individual basis and will be partially based on considerations of the condition to be treated and the desired outcome. A person skilled in the art can determine the effective amount by using the aforementioned factors such as individual basis and without exceeding routine experimentation.

When the pharmaceutical composition is used, a safe and effective amount of the immunoconjugate is administered to a mammal, with the safe and effective amount typically of at least about 10 µg/kg body weight, and in most cases not more than about 50 mg/kg body weight, preferably about 10 µg/kg body weight to about 20 µg/kg body weight. Without doubt, the specific dose should also take factors such as the route of administration and the patient's health status into consideration, all of which fall within the area of expertise of skilled physicians.

The present invention provides the use of the aforementioned pharmaceutical composition in the preparation of a medicament for preventing and/or treating a disease associated with abnormal BDCA2 expression or function. Preferably, the disease associated with abnormal BDCA2 expression or function is an inflammatory disease or an autoimmune disease.

### Applications

The present invention also provides uses of the antibody, antibody-drug conjugate (ADC), recombinant protein, and/or immune cell of the present invention, for example, for preparing a diagnostic agent or for preparing a medicament.

Preferably, the medicament is for preventing and/or treating a disease associated with abnormal BDCA2 expression or function.

In the present invention, the disease associated with abnormal BDCA2 expression or function is a conventional disease in the art associated with abnormal BDCA2 expression or function. Preferably, the disease associated with abnormal BDCA2 expression or function is an inflammatory disease or an autoimmune disease, including but not limited to systemic lupus erythematosus, cutaneous lupus erythematosus, discoid lupus, lupus nephritis, cutaneous lupus, rheumatoid arthritis, inflammatory bowel disease, systemic sclerosis (scleroderma), psoriasis, type I diabetes, dermatomyositis and polymyositis, or a combination thereof.

### Main Advantages of the Present Invention

(1) The murine anti-BDCA2 antibody of the present invention exhibits excellent affinity, superior to the positive control antibody Litifilimab; the humanized and modified anti-BDCA2 antibody also demonstrates higher affinity.
(2) The BDCA2 antibody of the present invention is capable of effectively inhibiting CpG-A-stimulated secretion of IFNα and IgM from PBMCs, showing potential as a therapeutic agent for autoimmune diseases.

The present invention is further described below in conjunction with specific examples. It should be understood that these examples are intended only to illustrate the present invention, rather than limiting the scope of the present invention. In the following examples, experimental methods without specified detailed conditions are generally carried out under conventional conditions as described in Sambrook et al., Molecular Cloning: A Laboratory Manual (New York: Cold Spring Harbor Laboratory Press, 1989), or as recommended by the manufacturers. Unless otherwise stated, percentages and parts are calculated by weight.

### Example 1 Antigen Immunization of Animals and Preparation and Screening of Hybridomas

### 1.1 Preparation of Antigen Protein and Positive Control Antibody

The sequence of the human BDCA2 extracellular domain (BDCA2-ECD) used as the antigen was obtained from the UniProt database (Entry: Q8WTT0), with the amino acid sequence shown in SEQ ID NO: 1. A 6×His tag was added to its C-terminus, and it was constructed into the pcDNA 3.4 expression vector. After transfection into HEK-293F cells and expression for 5 days, the cell culture supernatant was collected and purified to obtain BDCA2-his protein. Similarly, the 6×His tag was replaced with the Fc sequence of human IgG1, and after transfection into HEK-293F cells and expression purification, BDCA2-Fc protein was obtained.

The sequences of the heavy and light chains of the positive control antibody Litifilimab (BIIB059, 24F4A) were obtained from patent CN111961134A. After gene synthesis of the heavy chain variable region and light chain variable region of this antibody, they were recombined with the human heavy chain IgG1 and light chain kappa constant regions, respectively, and each was constructed into the pcDNA 3.4 expression vector, expressed using the HEK-293F system, and purified by Protein A affinity chromatography.

### 1.2 Mouse Immunization

Balb/c mice were routinely immunized with human BDCA2-His protein (self-prepared, purity >95%) expressed in mammalian 293F cells. On Day 1, soluble human BDCA2-His protein emulsified with Freund's complete adjuvant was administered to Balb/c mice via subcutaneous multi-point injection (100 µg human BDCA2-His/mouse/0.5 mL). On day 14, soluble human BDCA2-His protein emulsified with Freund's incomplete adjuvant was subcutaneously injected into Balb/c mice (50 µg human BDCA2-His/mouse/0.5 mL). On day 28, soluble human BDCA2-His protein emulsified with Freund's incomplete adjuvant was subcutaneously injected into Balb/c mice (50 µg human BDCA2-His/mouse/0.5 mL). Three weeks later, a booster dose of soluble human BDCA2-His protein (50 µg/mouse/0.2 mL) was administered via intraperitoneal injection. After 3-4 days, the mouse spleen was harvested for fusion experiments.

### 1.3 Preparation and Screening of Hybridoma Cells

Three to four days after the final immunization, mouse splenocytes were fused with mouse myeloma cells SP2/0 using a conventional hybridoma technique with PEG. The fused cells were uniformly suspended in complete medium, which consisted of RPMI1640-GLUMAX supplemented with 1% Penicillin-Streptomycin, 20% FBS (fetal bovine serum), and 1× HAT. The fused cells were plated at 3*10⁴ cells/ 200 µL/well across a total of 60 96-well culture plates for cultivation. After 7-12 days, the supernatant was harvested, and hybridoma wells positive for human BDCA2 binding activity were screened by ELISA. The method for screening hybridoma wells positive for human BDCA2 binding activity by ELISA is as follows: dilute BDCA2-Fc to 1 µg/mL with PBS buffer, add 100 µL/well to the plate, and incubate overnight at 4°C; the next day, discard the supernatant, add 5% skim milk and block at 37°C for 1 hour, wash the plate 3 times with PBST for later use; add the collected hybridoma supernatant sequentially to the blocked plate, 100 µL/well, and incubate at 37°C for 1 hour; wash the plate 3 times with PBST, add HRP-labeled goat anti-mouse IgG secondary antibody, and incubate at 37°C for 30 minutes; after washing the plate 3 times with PBST, remove residual droplets as much as possible on absorbent paper, add 100 µL of TMB to each well, and incubate at room temperature (20±5°C) protected from light for 5 minutes; add 50 µL of 2M H₂SO₄ stop solution to each well to terminate the substrate reaction, and read the OD value at 450 nm with a microplate reader to analyze the binding ability of the test antibody to the target antigen BDCA2. The hybridoma cell lines obtained through screening and expansion in complete medium containing serum were centrifuged and replaced with serum-free culture medium Hybridoma-SFM medium to achieve a cell density of 1-2×10⁶/mL, cultured under 8% CO₂ at 37°C for 1 week, centrifuged to collect the culture supernatant, purified by Protein G affinity chromatography to obtain anti-human BDCA2 monoclonal antibody protein, and through screening, a total of 30 hybridoma cell lines were obtained.

### 1.4 Binding Capacity of Murine Antibodies to Human BDCA2-Fc Protein

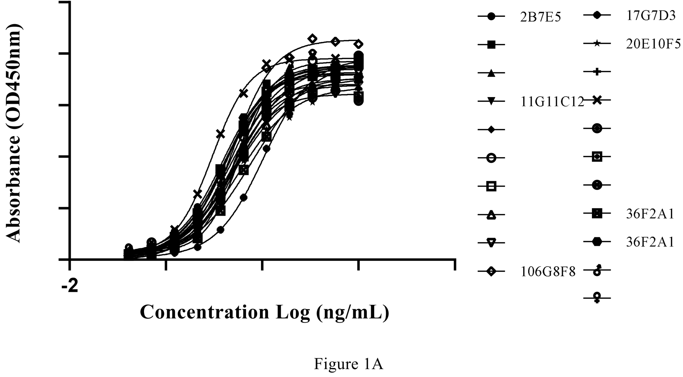

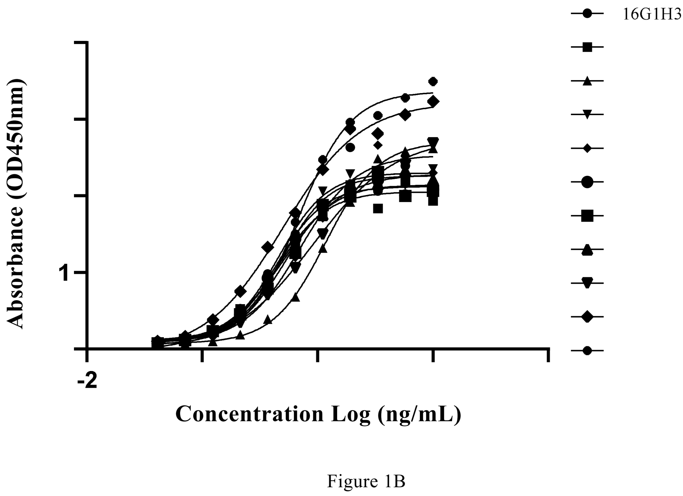

The binding capacity of murine antibodies to human BDCA2-Fc protein was determined by enzyme-linked immunosorbent assay (ELISA). The specific method is as follows: BDCA2-Fc protein was diluted to 1 µg/mL with PBS buffer and added to the plate at 100 µL/well, followed by incubation overnight at 4°C; the plate was then blocked with 5% skim milk and incubated at 37°C for 1 hour; after washing the plate three times with PBST, the laboratory-prepared anti-human BDCA2 murine antibodies were serially diluted 11-fold at 3-fold increments from 10 µg/mL using 1% BSA-PBS buffer, with 1% BSA-PBS as a blank control, and added to the pre-coated BDCA2-Fc plate at 100 µL/well, followed by incubation at 37°C for 1 hour; the plate was washed three times with PBST, and HRP-labeled goat anti-mouse IgG secondary antibody was added and incubated at 37°C for 3 minutes; after washing the plate three times with PBST, residual droplets were thoroughly removed by blotting on absorbent paper, and 100 µL of TMB was added to each well, followed by incubation at room temperature (20±5°C) in the dark for 5 minutes; 50 µL of 2M H₂SO₄ stop solution was added to each well to terminate the substrate reaction, and the OD value was measured at 450 nm using a microplate reader to analyze the binding capacity of the test antibody to the antigen human BDCA2-Fc. The obtained data were fitted and analyzed using GraphPad Prism 9 software, and the results are shown in Figures 1A-1B.

As shown in Figures 1A-1B, most murine antibodies exhibited good binding activity to the target antigen BDCA2-Fc, with EC50 values as shown in Tables 1-2.

**Table 1:**

| Antibody | EC50 (ng/mL) | Antibody | EC50 (ng/mL) |
|---|---|---|---|
| 2B7E5 | 14.69 | 17G7D3 | 107.2 |
| 2E3F6 | 18.85 | 20E10F5 | 32.65 |
| 2D12H3 | 21.86 | 21B10A3 | 22.51 |
| 11G11C12 | 16.86 | 21E1A3 | 9.28 |
| 27G9G9 | 24.19 | 21E5C8 | 25.41 |
| 39E3B2 | 23.59 | 26C3F1 | 28.01 |
| 44H3A7 | 16.44 | 35E12F1 | 31.31 |
| 52G1A3 | 28.15 | 36F2A1 | 63.8 |
| 52H10D11 | 28.2 | 39F4D1 | 15.79 |
| 106G8F8 | 25.34 | 55G10A9 | 26.19 |
| | | 24F4A | 34.61 |

**Table 2:**

| Antibody | EC50 (ng/mL) |
|---|---|
| 16G1H3 | 21.34 |
| 23C10A6B5 | 20.14 |
| 36A6C5E2 | 138.3 |
| 39F6C9 | 24.55 |
| 40H1F3G8 | 49.29 |
| 48A5H1F10 | 19.54 |
| 52E5D3 | 26.54 |
| 136B4H2E3 | 26.51 |
| 144A4G1A1 | 83.4 |
| 146D6A9E5 | 25.33 |
| 24F4A | 46.27 |

### 1.5 Determination of Binding Affinity of Murine Antibodies to Stable Transfectant 293FT-BDCA2

In this example, the binding affinity of murine antibodies to the target cells 293FT-BDCA2 was determined by fluorescence-activated cell sorting (FACS).

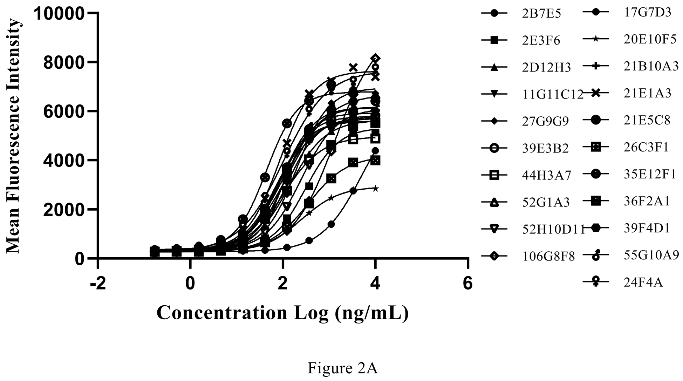

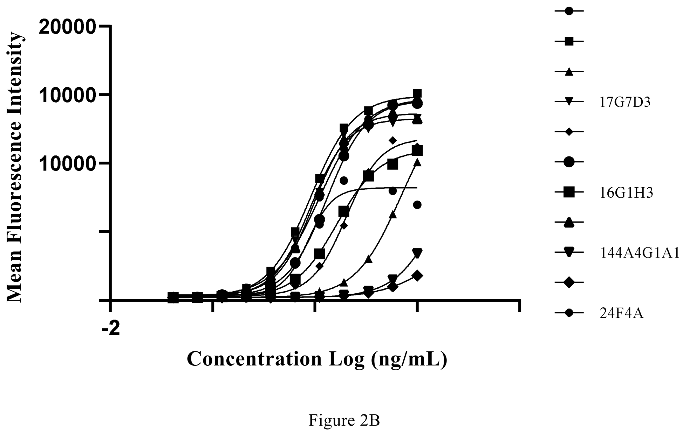

In this experiment, 293FT-BDCA2 (an engineered cell line constructed in the laboratory using lentivirus to highly express human BDCA2) was used as the target cells. The cells were counted, washed once with 1% BSA-PBS, plated at 2×10⁵ cells per well with 100 µL per well, centrifuged to remove the supernatant. The anti-human BDCA2 murine antibody was serially diluted 11 gradients at 3-fold increments from 10 µg/mL using 1% BSA-PBS, with 1% BSA-PBS as a blank control. Then, 100 µL of each dilution was added to resuspend the 293FT-BDCA2 cells in the wells, followed by incubation at 4°C for 1 hour. The cells were washed twice with PBS to remove unbound murine antibodies, then resuspended in 100 µL per well of 1:500 diluted PE-labeled anti-mouse secondary antibody (purchased from Jackson, catalog number 715116150), and incubated at 4°C for 1 hour. The cells were washed twice with PBS to remove unbound secondary antibodies, and finally resuspended in 200 µL PBS. The binding affinity of the murine antibodies to the cells was determined by flow cytometry, and the obtained data were fitted and analyzed using GraphPad Prism 9 software. The results are shown in Figure 2A to Figure 2B.

The results indicate that most murine antibodies can specifically bind to BDCA2 on the surface of 293FT cells, with EC50 values as shown in Table 3 and Table 4.

**Table 3:**

| Antibody | EC50 (ng/mL) | Antibody | EC50 (ng/mL) |
|---|---|---|---|
| 2B7E5 | 72.08 | 17G7D3 | 5251 |
| 2E3F6 | 306.4 | 20E10F5 | 257.3 |
| 2D12H3 | 139.5 | 21B10A3 | 124.7 |
| 11G11C12 | 87.09 | 21E1A3 | 85.94 |
| 27G9G9 | 198.5 | 21E5C8 | 108.2 |
| 39E3B2 | 78.75 | 26C3F1 | 78.28 |
| 44H3A7 | 103.1 | 35E12F1 | 44.23 |
| 52G1A3 | 114.8 | 36F2A1 | 349.7 |
| 52H10D11 | 248.8 | 39F4D1 | 135.6 |
| 106G8F8 | 1348 | 55G10A9 | 92.82 |
| | | 24F4A | 103.4 |

**Table 4:**

| Antibody | EC50 (ng/mL) |
|---|---|
| 16G1H3 | 72.7 |
| 23C10A6B5 | 83.53 |
| 36A6C5E2 | 4787 |
| 39F6C9 | 81.91 |
| 40H1F3G8 | 410.1 |
| 48A5H1F10 | 172.6 |
| 52E5D3 | 272.3 |
| 136B4H2E3 | 94.27 |
| 144A4G1A1 | 31337 |
| 146D6A9E5 | 17724 |
| 24F4A | 124.2 |

### Example 2 Humanization of Murine Anti-Human BDCA2 Monoclonal Antibodies 2.1 Determination of Variable Region Sequences of Murine Anti-Human BDCA2 Monoclonal Antibodies

Hybridoma clone 23C10A6B5 was selected as candidate antibodies. Total RNA was extracted from the corresponding hybridoma monoclonal cell lines using Trizol (purchased from Life Technologies), and mRNA was reverse transcribed into cDNA using a reverse transcription kit (purchased from Takara). PCR was performed using combinatorial primers reported in the literature (Antibody Engineering, Volume 1, Edited by Roland Kontermann and Stefan Dübel, primer sequences from page 323). The obtained PCR products were sequenced and analyzed via the Kabat database to confirm that the obtained sequences were variable region sequences of murine antibodies. The sequence information is as follows:
The heavy chain variable region gene sequence is 354 bp in length, encoding 118 amino acid residues. The nucleotide sequence is shown in SEQ ID NO: 2, and the amino acid sequence is shown in SEQ ID NO: 3.

The light chain variable region gene sequence is 321 bp in length, encoding 107 amino acid residues. The nucleotide sequence is shown in SEQ ID NO: 4, and the amino acid sequence is shown in SEQ ID NO: 5.

### 2.2 Construction and Activity Validation of Human-Mouse Chimeric Antibody

The murine heavy chain variable region and light chain variable region sequences (SEQ ID NO: 3 and SEQ ID NO: 5) obtained in Example 2.1 were respectively linked to the human IgG1 heavy chain constant region and the kappa-type light chain constant region to construct the human-mouse chimeric antibody C-23C10. Its binding activity to the antigen BDCA2 and its inhibitory effect on CpG-A-induced IFNα secretion by PBMCs were validated.

The method for detecting binding activity was as described in Example 1.4, with the distinction that the coated antigen BDCA2-Fc was replaced with BDCA2-his at a coating concentration of 1 µg/mL, and the secondary antibody was replaced with anti-human Fc HRP (SinoBiological, Catalog No. SSA001) at a working concentration of 1:3000.

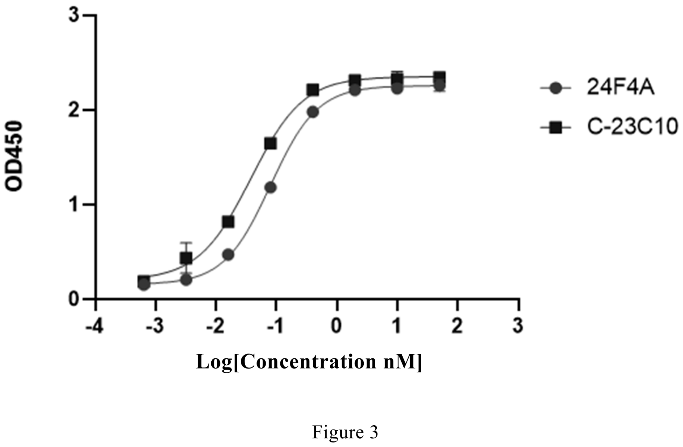

The experimental results are shown in Figure 3. The binding activity of C-23C10 to the BDCA2 antigen was superior to that of the positive control 24F4A.

CpG-A (CpG ODNs Class A) is an oligodeoxynucleotide containing CpG palindromic sequences, which can stimulate pDCs to secrete large amounts of IFNα. In this example, PBMCs were used to detect the inhibitory effect of candidate antibodies on CpG-A-induced IFNα secretion by PBMCs.

CpG-A was diluted to 8 µg/mL (final concentration 2 µg/mL) in RPMI 1640 + 3% FBS medium and added to a flat-bottom 96-well plate at 50 µL per well. Antibodies were diluted to 16 nM in RPMI 1640 + 3% FBS medium, followed by 5-fold serial dilution (final concentration 4 nM, 5-fold dilution), and added to the CpG-A-containing wells at 50 µL per well. CpG-A and antibodies were co-incubated at 37°C in a 5% CO₂ incubator for 1 hour. PBMCs were centrifuged and counted, adjusted to the appropriate cell density, and seeded into the above 96-well plate at 500,000 cells per well in 100 µL medium, followed by incubation overnight at 37°C in a 5% CO₂ incubator. The cell supernatant was collected by centrifugation, and the content of H-IFNα in the supernatant was measured.

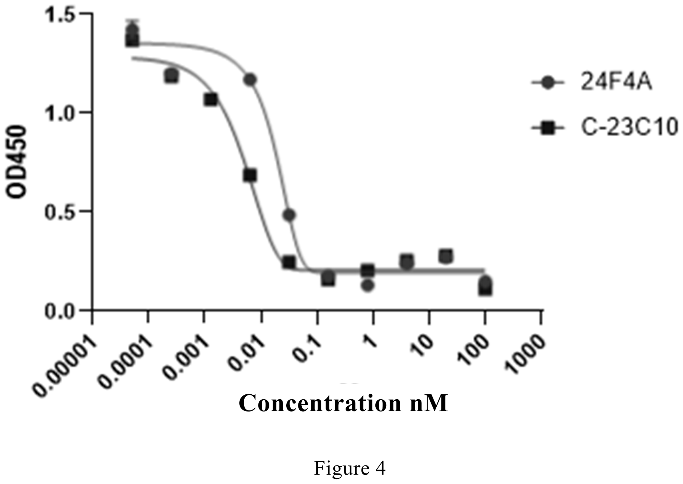

The experimental results are shown in Figure 4. C-23C10 effectively inhibited CpG-A-induced IFNα secretion by PBMCs, and its activity was superior to that of the positive control 24F4.

### 2.3 Humanization of Murine Anti-Human BDCA2 Monoclonal Antibody

The amino acid sequences of the heavy and light chain variable regions of 23C10A6B5 were analyzed, and three complementarity-determining regions (CDRs) and four framework regions (FRs) were identified according to Kabat rules. The amino acid sequences of the heavy chain complementarity-determining regions are:
HCDR1: SYIIH (SEQ ID NO: 6),
HCDR2: TIYPGNGDTSYNQKFKG (SEQ ID NO: 7), and
HCDR3: MGDNEYFDY (SEQ ID NO: 8); the amino acid sequences of the light chain complementarity-determining regions are:
   LCDR1: RASGNIHNYLA (SEQ ID NO: 9),
   LCDR2: DAETLAD (SEQ ID NO: 10), and
   LCDR3: QHFWSTPYT (SEQ ID NO: 11).

By performing homology comparison with human IgG germline sequences (Germline) in NCBI IgBlast, IGHV1-46*01 was selected as the heavy chain CDR grafting template and IGKV1-NL1*01 as the light chain CDR grafting template. The CDR regions of the 23C10A6B5 antibody were grafted onto the selected humanized templates, replacing the CDR regions of the human templates. The heavy chain variable region was then recombined with the human IgG1 constant region, and the light chain variable region was recombined with the human kappa chain constant region. Based on the three-dimensional structure of the antibody, back mutations were performed on buried residues, residues directly interacting with the CDR regions, and residues significantly influencing the conformation of the antibody's VL and VH. Multiple humanized antibodies were obtained, and through affinity screening, the amino acid sequence of the heavy chain variable region (SEQ ID NO:12) and the light chain variable region (SEQ ID NO:13) of the humanized antibody 230 were determined.

### Example 3 Affinity Maturation of Humanized Antibody 230

Using AlphaFold2 software, complex structure prediction and interaction interface analysis were performed for the 230 antibody and BDCA2-his antigen. A mutant library was constructed by introducing single-point saturation mutations at key interaction sites. Through ELISA affinity screening, mutation sites that could enhance the affinity of the 230 antibody were identified. The experimental method referred to Example 1.4, with the distinction that the coating antigen was changed from BDCA2-Fc to BDCA2-his, at a coating concentration of 0.1 µg/mL (i.e., 10 ng per well), and the secondary antibody was replaced with anti-human Fc HRP (SinoBiological, Cat. No. SSA001) at a working concentration of 1:3000.

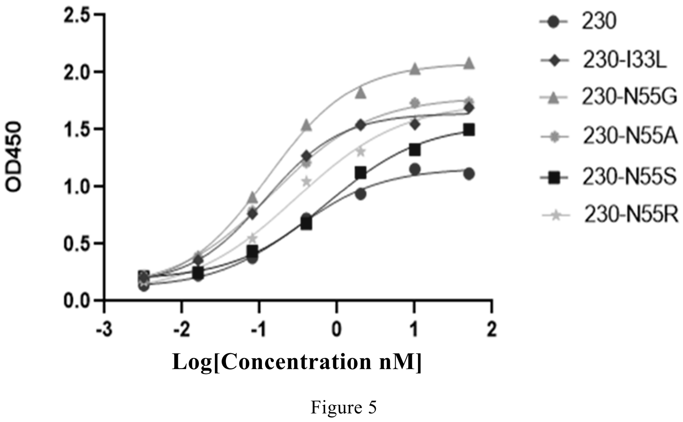

The results are shown in Figure 5. The experimental results indicate that
the mutation of I to L in the heavy chain CDR1: SYIIH (SEQ ID NO: 6) resulted in SYLIH (SEQ ID NO: 14); and the mutation of N in the heavy chain CDR2: TIYPGNGDTSYNQKFKG (SEQ ID NO: 7) to G, A, S, and R, respectively, yielded
TIYPGGGDTSYNQKFKG (SEQ ID NO: 15),
TIYPGAGDTSYNQKFKG (SEQ ID NO: 16),
TIYPGSGDTSYNQKFKG (SEQ ID NO: 17),
TIYPGRGDTSYNQKFKG (SEQ ID NO: 18) (corresponding to antibodies 230-I33L, 230-N55G, 230-N55A, 230-N55S, and 230-N55R, respectively), all of which effectively enhanced the affinity of antibody 230.

Furthermore, mutation of D in the light chain CDR2: DAETLAD (SEQ ID NO: 10) to I or E resulted in DAETLAI (SEQ ID NO: 19) and DAETLAE (SEQ ID NO: 20), respectively. These mutants maintained affinity comparable to that of 230, while the expression level increased by an average of over 30%. The remaining saturation mutants exhibited both affinity and expression levels comparable to those of the parental 230 antibody.

Selected mutations from the aforementioned CDR regions were combined and linked to the constant region of the IgG1 subtype to construct antibodies 230IL-DI, 230IL-DE, 230IL-NG-DI, 230IL-NG-DE, 230IL-NR-DI, 230IL-NR-DE, 230NG-DI, 230NG-DE, 230NR-DI, and 230NR-DE. Among them, antibodies 230IL-DI and 230IL-DE share the same heavy chain, with the heavy chain variable region sequence as shown in SEQ ID NO: 21; antibodies 230IL-NG-DI and 230IL-NG-DE share the same heavy chain, with the heavy chain variable region sequence as shown in SEQ ID NO: 22; antibodies 230IL-NR-DI and 230IL-NR-DE share the same heavy chain, with the heavy chain variable region sequence as shown in SEQ ID NO: 23; antibodies 230NG-DI and 230NG-DE share the same heavy chain, with the heavy chain variable region sequence as shown in SEQ ID NO: 24; antibodies 230NR-DI and 230NR-DE share the same heavy chain, with the heavy chain variable region sequence as shown in SEQ ID NO: 25. Antibodies 230IL-DI, 230IL-NG-DI, 230IL-NR-DI, 230NG-DI, and 230NR-DI share the same light chain, with the light chain variable region sequence as shown in SEQ ID NO: 26; antibodies 230IL-DE, 230IL-NG-DE, 230IL-NR-DE, 230NG-DE, and 230NR-DE share the same light chain, with the light chain variable region sequence as shown in SEQ ID NO: 27.

The affinity of antibodies 230IL-DI, 230IL-DE, 230IL-NG-DI, 230IL-NG-DE, 230IL-NR-DI, 230IL-NR-DE, 230NG-DI, 230NG-DE, 230NR-DI, and 230NR-DE for BDCA2-his antigen protein was evaluated according to the aforementioned ELISA experimental method.

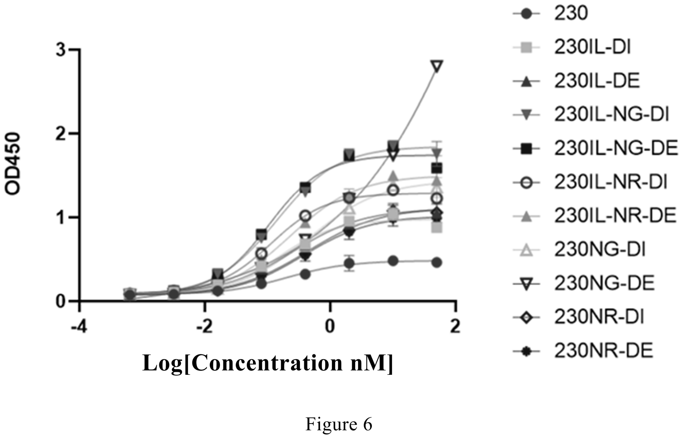

The results are shown in Figure 6. The experimental results indicate that the affinity of all ten aforementioned mutant strains for the antigen protein is higher than that of the parental monoclonal antibody 230.

### Example 4 Determination of Affinity Dissociation Constant (KD) Value

For the heavy chain Fc regions of antibodies 230IL-NG-DE and 230-NG-DE (also referred to as 230NG-DE) from Example 3, mutations M428L/N434S and G236A/S239D/I332E were simultaneously introduced according to patents CN200880123009.4 and CN200480018985.5, to construct antibody 237011 (heavy chain sequence as shown in SEQ ID NO: 28, light chain variable region sequence as shown in SEQ ID NO: 27) and antibody 2391 (heavy chain sequence as shown in SEQ ID NO: 29, light chain variable region sequence as shown in SEQ ID NO: 27).

The kinetic parameters of binding and dissociation between candidate antibody proteins and antigen BDCA2-his were determined using a Biacore 8K molecular interaction analyzer via the capture method. Antibodies were diluted to 1 µg/mL in 1× HBS-N buffer containing 5 mM CaCl₂, 0.02% Tween 20, pH 7.0, and captured using a Protein A chip. The antigen was diluted in 1× HBS-N buffer containing 5 mM CaCl₂, 0.02% Tween 20, pH 7.0, and six concentration gradients with a maximum concentration of 12.5 nM were set for binding with the antibodies. Dissociation was carried out in 1× HBS-N buffer containing 5 mM CaCl₂, 0.02% Tween 20, pH 7.0.

The experimental results are shown in Table 5. The affinity of candidate antibodies 237011 and 2391 for the antigen BDCA2-his is higher than that of the positive control antibody 24F4A.

**Table 5: Affinity Dissociation Constant**

| Mobile Phase | Stationary Phase | KD (M) | ka (1/Ms) | Kd (1/s) |
|---|---|---|---|---|
| BDCA2-his | 237011 | 2.59E-11 | 2.52E+07 | 6.52E-04 |
| BDCA2-his | 2391 | 3.57E-11 | 2.44E+07 | 8.74E-04 |
| BDCA2-his | 230 | 7.68E-11 | 1.70E+07 | 1.31E-03 |
| BDCA2-his | 24F4A | 8.70E-11 | 1.39E+06 | 1.21E-04 |

| | | | | |
|---|---|---|---|---|
| Note: KD is the affinity constant; ka is the association rate constant; kd is the dissociation rate constant. | | | | |

### Example 5 Inhibitory Effect on CpG-A-Induced IFNα Secretion by PBMCs

The inhibitory effect of humanized candidate antibodies on CpG-A-induced IFNα secretion by PBMCs was validated, following the experimental method described in Example 2.2.

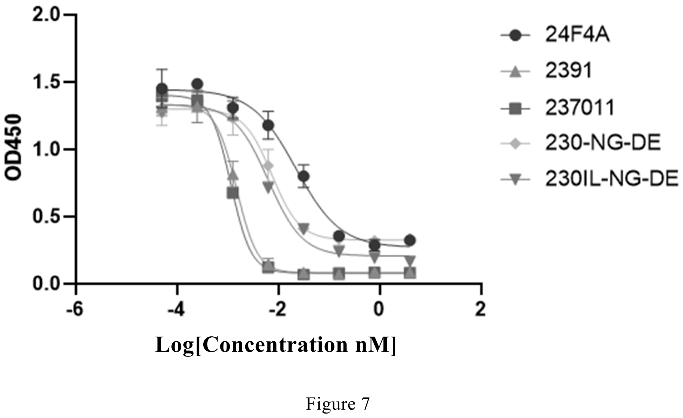

The experimental results are shown in Figure 7. Candidate antibodies 237011, 2391, 230IL-NG-DE, and 230-NG-DE all effectively inhibited CpG-A-induced IFNα secretion by PBMCs, and their activities were significantly superior to that of the positive control antibody 24F4A.

### Example 6 Inhibitory Effect on CpG-A-Induced IgM Secretion by PBMCs

To validate the inhibitory effect of humanized candidate antibodies on CpG-A-induced IgM secretion by PBMCs, the experimental method was as follows: PBMC cells were resuspended in RPMI 1640 + 10% FBS medium, adjusted to a cell density of 5×10⁶/ml, and 100 µL per well was added to a 3599 plate. Then, 50 µL of CPG-A (CpG2216) was added to each well at a final concentration of 2 µg/mL; antibodies were diluted with medium, and 50 µL was added to each well at a final concentration of 3000 nM. First, 100 µL of cells and 50 µL of antibody were co-incubated at 37°C, 5% CO₂ for 1 hour, followed by the addition of 50 µL of CPG-A, and then incubated at 37°C, 5% CO₂. After 7 days of incubation, the cell supernatant was collected by centrifugation, and the IgM content in the supernatant was measured.

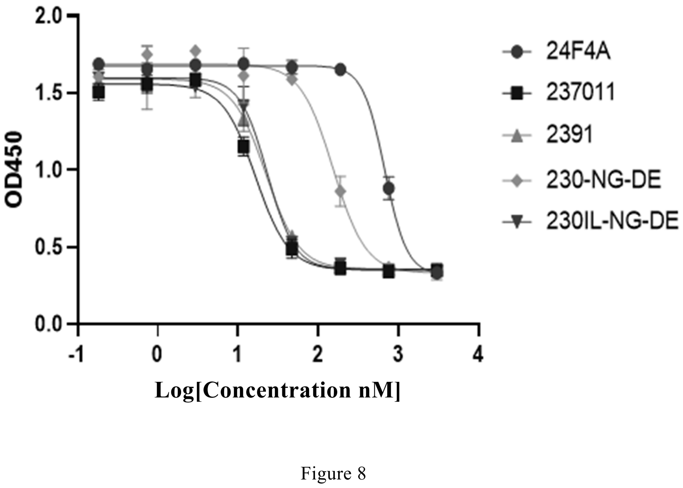

The experimental results are shown in Figure 8. Candidate antibodies 237011, 2391, 230IL-NG-DE, and 230-NG-DE all effectively inhibited CpG-A-induced IgM secretion by PBMCs, and their activities were significantly superior to that of the positive control antibody 24F4A.

### Example 7 Inhibitory Effect on IFNα in Mice

To validate the inhibitory effect of humanized candidate antibodies on IFNα secretion in mice, and since the candidate antibodies do not cross-react with homologous proteins of BDCA2 in mice, reconstructed humanized immune system mice established by injecting human huCD34+ embryonic stem cells (HuHSC-NCG immune system humanized mice, purchased from Jiangsu GemPharmatech Co., Ltd.) were used for in vivo animal experiments.

The experimental protocol was as follows: On Day 0, the test mice were weighed and randomly divided into three groups based on body weight: a model group (PBS administration group), a 24F4A administration group, and a 237011 administration group. That afternoon, each group was administered according to the protocol in Table 6 via intraperitoneal injection. Eighteen hours after administration (Day 1), the three groups of mice were injected with CpG-A via tail vein at a dose of 500 µg/250 µL per mouse. Twenty-four hours after CpG-A injection, blood was collected from the retro-orbital venous plexus, and after coagulation, serum was obtained by centrifugation. The concentration of human IFNα in the serum was measured by ELISA.

**Table 6: Group Administration Protocol**

| **Group** | | **Dose** | **Administration Route** | **Administration Frequency/Times** | **Number of Animals** |
|---|---|---|---|---|---|
| Group 1 | CpG-A + PBS | N/A | Intraperitoneal injection | Single administration | 6 |
| Group 2 | CpG-A + 24F4A | 10 mg/kg | Intraperitoneal injection | Single administration | 7 |
| Group 3 | CpG-A + 237011 | 10 mg/kg | Intraperitoneal injection | Single administration | 7 |

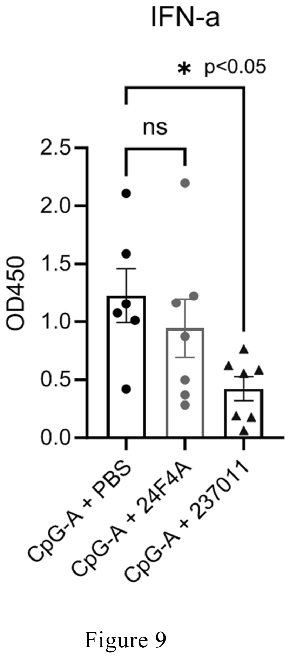

The experimental results are shown in Figure 9. The 237011 administration group demonstrated significantly superior inhibition of IFNα activity in mice compared to the 24F4A administration group.

**The sequences of the present invention are as follows:**
Human BDCA2 extracellular domain (BDCA2-ECD) SEQ ID NO: 1 
NFMYSKTVKRLSKLREYQQYHPSLTCVMEGKDIEDWSCCPTPWTSFQSSCYFISTGMQSWT
KSQKNCSVMGADLVVINTREEQDFIIQNLKRNSSYFLGLSDPGGRRHWQWVDQTPYNENV
TFWHSGEPNNLDERCAIINFRSSEEWGWNDIHCHVPQKSICKMKKIYI
23C10A6B5 heavy chain variable region nucleotide sequence SEQ ID NO: 2 
CAGGTGCAACTACAGCAGCCTGGGGCTGACCTGGTGAAGCCTGGGGCCTCAGTGATGA
TGTCCTGCAAGGCTTCTGGATACACATTTACCAGTTACATTATTCACTGGGTAAAACAG
ACACCTGGACAGGGCCTGGAATGGATTGGAACTATTTATCCAGGAAATGGTGATACTTC
CTACAATCAGAAATTCAAAGGCAAGGCCACATTGACTGCAGACAAATCCTCCAGCACA
GCCTACATGCAGCTCAGCAGCCTGACATCTGAGGACTCTGCGGTCTATTACTGTGCAAG
AATGGGGGATAACGAATACTTTGACTACTGGGGCCAAGGAACCACTCTCACAGTCTCCT
CA
23C10A6B5 heavy chain variable region amino acid sequence SEQ ID NO: 3 
QVQLQQPGADLVKPGASVMMSCKASGYTFTSYIIHWVKQTPGQGLEWIGTIYPGNGDTSY
NQKFKGKATLTADKSSSTAYMQLSSLTSEDSAVYYCARMGDNEYFDYWGQGTTLTVSS
23C10A6B5 light chain variable region nucleotide sequence SEQ ID NO: 4 
GACATCCAGATGACTCAGTCTCCAGCCTCCCTATCTGCATCTGTGGGAGAAACTGTCAC
CATCACATGTCGAGCAAGTGGAAATATTCACAATTATTTAGCATGGTATCAGCAGAAAC
AGGGAAAATCTCCTCAGCTCCTGGTCTATGATGCAGAAACCTTAGCAGATGGTGTGCCA
TCAAGGTTCAGTGGCAGTGGATCCGGAACACAATATTCTCTCAATATCAACAGCCTGCA
GCCTGAAGATTTTGGGAGTTTTTACTGTCAACATTTTTGGAGTACTCCGTACACGTTCGG
AGGGGGGACCAAGCTGGAAATAAAA
23C10A6B5 light chain variable region amino acid sequence SEQ ID NO: 5 
DIQMTQSPASLSASVGETVTITCRASGNIHNYLAWYQQKQGKSPQLLVYDAETLADGVPSR
FSGSGSGTQYSLNINSLQPEDFGSFYCQHFWSTPYTFGGGTKLEIK
HCDR1 of 23C10A6B5: SYIIH (SEQ ID NO: 6)
HCDR2 of 23C10A6B5: TIYPGNGDTSYNQKFKG (SEQ ID NO: 7)
HCDR3 of 23C10A6B5: MGDNEYFDY (SEQ ID NO: 8)
LCDR1 of 23C10A6B5: RASGNIHNYLA (SEQ ID NO: 9)
LCDR2 of 23C10A6B5: DAETLAD (SEQ ID NO: 10)
LCDR3 of 23C10A6B5: QHFWSTPYT (SEQ ID NO: 11)
Heavy chain variable region of humanized antibody 230 SEQ ID NO: 12 
QVQLVQSGAEVKKPGASVKVSCKASGYTFTSYIIHWVRQAPGQGLEWMGTIYPGNGDTSY
NQKFKGRVTMTRDTSTSTVYMELSSLRSEDTAVYYCARMGDNEYFDYWGQGTLVTVSS
Light chain variable region of humanized antibody 230 SEQ ID NO: 13

DIQMTQSPSSLSASVGDRVTITCRASGNIHNYLAWYQQKPGKAPKLLLYDAETLADGVPSR
FSGSGSGTDYTLTISSLQPEDFATYYCQHFWSTPYTFGGGTKVEIK

| | | |
|---|---|---|
| 230 antibody-IL heavy chain CDR1 | SEQ ID NO: | 14 SYLIH |
| 230 antibody-NG heavy chain CDR2 | SEQ ID NO: | 15 TIYPGGGDTSYNQKFKG |
| 230 antibody-NA heavy chain CDR2 | SEQ ID NO: | 16 TIYPGAGDTSYNQKFKG |
| 230 antibody-NS heavy chain CDR2 | SEQ ID NO: | 17 TIYPGSGDTSYNQKFKG |
| 230 antibody-NR heavy chain CDR2 | SEQ ID NO: | 18 TIYPGRGDTSYNQKFKG |
| 230 antibody-DI light chain CDR2 | SEQ ID NO: | 19 DAETLAI |
| 230 antibody-DE light chain CDR2 | SEQ ID NO: | 20 DAETLAE |
| 230IL-DI heavy chain variable region | SEQ ID NO: | 21 |

QVQLVQSGAEVKKPGASVKVSCKASGYTFTSYLIHWVRQAPGQGLEWMGTIYPGNGDTS
YNQKFKGRVTMTRDTSTSTVYMELSSLRSEDTAVYYCARMGDNEYFDYWGQGTLVTVSS

230IL-NG-DI heavy chain variable region SEQ ID NO: 22 
QVQLVQSGAEVKKPGASVKVSCKASGYTFTSYLIHWVRQAPGQGLEWMGTIYPGGGDTS
YNQKFKGRVTMTRDTSTSTVYMELSSLRSEDTAVYYCARMGDNEYFDYWGQGTLVTVSS
230IL-NR-DI heavy chain variable region SEQ ID NO: 23 
QVQLVQSGAEVKKPGASVKVSCKASGYTFTSYLIHWVRQAPGQGLEWMGTIYPGRGDTSY
NQKFKGRVTMTRDTSTSTVYMELSSLRSEDTAVYYCARMGDNEYFDYWGQGTLVTVSS
230NG-DI heavy chain variable region SEQ ID NO: 24 
QVQLVQSGAEVKKPGASVKVSCKASGYTFTSYIIHWVRQAPGQGLEWMGTIYPGGGDTSY
NQKFKGRVTMTRDTSTSTVYMELSSLRSEDTAVYYCARMGDNEYFDYWGQGTLVTVSS
230NR-DI heavy chain variable region SEQ ID NO: 25 
QVQLVQSGAEVKKPGASVKVSCKASGYTFTSYIIHWVRQAPGQGLEWMGTIYPGRGDTSY
NQKFKGRVTMTRDTSTSTVYMELSSLRSEDTAVYYCARMGDNEYFDYWGQGTLVTVSS
230IL-DI light chain variable region SEQ ID NO: 26 
DIQMTQSPSSLSASVGDRVTITCRASGNIHNYLAWYQQKPGKAPKLLLYDAETLAIGVPSRF
SGSGSGTDYTLTISSLQPEDFATYYCQHFWSTPYTFGGGTKVEIK
230IL-DE light chain variable region SEQ ID NO: 27 
DIQMTQSPSSLSASVGDRVTITCRASGNIHNYLAWYQQKPGKAPKLLLYDAETLAEGVPSR
FSGSGSGTDYTLTISSLQPEDFATYYCQHFWSTPYTFGGGTKVEIK
237011 antibody heavy chain SEQ ID NO: 28 
QVQLVQSGAEVKKPGASVKVSCKASGYTFTSYLIHWVRQAPGQGLEWMGTIYPGGGDTS
YNQKFKGRVTMTRDTSTSTVYMELSSLRSEDTAVYYCARMGDNEYFDYWGQGTLVTVSS
ASTKGPSVFPLAPSSKSTSGGTAALGCLVKDYFPEPVTVSWNSGALTSGVHTFPAVLQSSGL
YSLSSVVTVPSSSLGTQTYICNVNHKPSNTKVDKKVEPKSCDKTHTCPPCPAPELLAGPDVF
LFPPKPKDTLMISRTPEVTCVVVDVSHEDPEVKFNWYVDGVEVHNAKTKPREEQYNSTYR
VVSVLTVLHQDWLNGKEYKCKVSNKALPAPEEKTISKAKGQPREPQVYTLPPSREEMTKN
QVSLTCLVKGFYPSDIAVEWESNGQPENNYKTTPPVLDSDGSFFLYSKLTVDKSRWQQGNV
FSCSVLHEALHSHYTQKSLSLSPGK
2391 antibody heavy chain SEQ ID NO: 29 
QVQLVQSGAEVKKPGASVKVSCKASGYTFTSYIIHWVRQAPGQGLEWMGTIYPGGGDTSY
NQKFKGRVTMTRDTSTSTVYMELSSLRSEDTAVYYCARMGDNEYFDYWGQGTLVTVSSA
STKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKKVEPKSCDKTHTCPPCPAPELLAGPDVFL
FPPKPKDTLMISRTPEVTCVVVDVSHEDPEVKFNWYVDGVEVHNAKTKPREEQYNSTYRV
VSVLTVLHQDWLNGKEYKCKVSNKALPAPEEKTISKAKGQPREPQVYTLPPSREEMTKNQ
VSLTCLVKGFYPSDIAVEWESNGQPENNYKTTPPVLDSDGSFFLYSKLTVDKSRWQQGNVF
SCSVLHEALHSHYTQKSLSLSPGK
HCDR1 of anti-BDCA2 antibody SYX₁IH, wherein X₁ is I or L (SEQ ID NO: 30)
HCDR2 of anti-BDCA2 antibody TIYPGX₂GDTSYNQKFKG, wherein X₂ is N or G or A or S or R (SEQ ID NO: 31)
LCDR2 of anti-BDCA2 antibody DAETLAX₃, wherein X₃ is I or E (SEQ ID NO: 32)

All publications mentioned in the present invention are incorporated in the present application by reference to the same extent as if each individual publication was individually indicated to be incorporated by reference. Furthermore, it should be understood that, after reading the above teachings of the present invention, those skilled in the art can make various changes or modifications to the present invention, and these equivalent forms shall also fall within the scope defined by the claims attached to the present application.

## Claims

1. An antibody or antigen-binding fragment thereof that binds to BDCA2, comprising a heavy chain variable region and a light chain variable region, the heavy chain variable region comprises heavy chain complementarity determining regions H-CDR1, H-CDR2, and H-CDR3, wherein
the amino acid sequence of H-CDR1 is as shown in SYX₁IH (SEQ ID NO: 30),
the amino acid sequence of H-CDR2 is as shown in TIYPGX₂GDTSYNQKFKG (SEQ ID NO: 31),
the amino acid sequence of H-CDR3 is as shown in MGDNEYFDY (SEQ ID NO: 8), and
the light chain variable region comprises light chain complementarity determining regions L-CDR1, L-CDR2, and L-CDR3; wherein,
the amino acid sequence of L-CDR1 is as shown in RASGNIHNYLA (SEQ ID NO: 9),
the amino acid sequence of L-CDR2 is as shown in DAETLAX₃ (SEQ ID NO: 32),
the amino acid sequence of L-CDR3 is as shown in QHFWSTPYT (SEQ ID NO: 11);
wherein, X₁ is I or L; X₂ is an amino acid selected from the group consisting of: N, G, A, S, R; and/or X₃ is an amino acid selected from the group consisting of: D, I, or E.

2. The antibody or antigen-binding fragment thereof according to claim 1, wherein X₁, X₂, and X₃ are selected from the group consisting of:
X₁ is I, X₂ is N, X₃ is D;
X₁ is L, X₂ is N, X₃ is I;
X₁ is L, X₂ is N, X₃ is E;
X₁ is L, X₂ is G, X₃ is I;
X₁ is L, X₂ is G, X₃ is E;
X₁ is L, X₂ is R, X₃ is I;
X₁ is L, X₂ is R, X₃ is E;
X₁ is I, X₂ is G, X₃ is I;
X₁ is I, X₂ is G, X₃ is E;
X₁ is I, X₂ is R, X₃ is I; or
X₁ is I, X₂ is R, X₃ is E.

3. The antibody or antigen-binding fragment thereof according to claim 1, wherein the heavy chain variable region comprises heavy chain complementarity determining regions H-CDR1, H-CDR2, and H-CDR3, wherein
the amino acid sequence of H-CDR1 is as shown in SYIIH (SEQ ID NO: 6),
the amino acid sequence of H-CDR2 is as shown in TIYPGNGDTSYNQKFKG (SEQ ID NO: 7),
the amino acid sequence of H-CDR3 is as shown in MGDNEYFDY (SEQ ID NO: 8), and
the light chain variable region comprises light chain complementarity determining regions L-CDR1, L-CDR2, and L-CDR3; wherein,
the amino acid sequence of L-CDR1 is as shown in RASGNIHNYLA (SEQ ID NO: 9),
the amino acid sequence of L-CDR2 is as shown in DAETLAD (SEQ ID NO: 10),
the amino acid sequence of L-CDR3 is as shown in QHFWSTPYT (SEQ ID NO: 11);
wherein any one of the aforementioned amino acid sequences further optionally comprises a derivative sequence that optionally undergoes addition, deletion, modification, and/or substitution of at least one amino acid and is capable of retaining BDCA2 binding affinity.

4. The antibody or antigen-binding fragment thereof that binds to BDCA2 according to claim 1, wherein the amino acid sequence of the heavy chain variable region of the antibody or antigen-binding fragment thereof that binds to BDCA2 is as shown in SEQ ID NO: 3, and the amino acid sequence of the light chain variable region is as shown in SEQ ID NO: 5; or the amino acid sequence of the heavy chain variable region of the antibody or antigen-binding fragment thereof that binds to BDCA2 is as shown in SEQ ID NO: 12, and the amino acid sequence of the light chain variable region is as shown in SEQ ID NO: 13; or the amino acid sequence of the heavy chain variable region of the antibody or antigen-binding fragment thereof that binds to BDCA2 is selected from SEQ ID NO: 21, 22, 23, 24, or 25, and the amino acid sequence of the light chain variable region is selected from SEQ ID NO: 26 or 27.

5. The antibody or antigen-binding fragment thereof according to claim 1, wherein the antibody is selected from the group consisting of: animal-derived antibody, chimeric antibody, humanized antibody, fully human antibody, or a combination thereof.

6. A recombinant protein, wherein the recombinant protein comprises:
(i) the antibody or antigen-binding fragment thereof that binds to BDCA2 according to any one of claims 1-5; and
(ii) optionally, a tag sequence that facilitates expression and/or purification.

7. A nucleotide molecule, wherein the nucleotide molecule encodes the antibody or antigen-binding fragment thereof that binds to BDCA2 according to any one of claims 1-5.

8. An expression vector, wherein the expression vector comprises the nucleotide molecule according to claim 7.

9. A host cell, wherein the host cell comprises the expression vector according to claim 8.

10. A method for preparing the antibody or antigen-binding fragment thereof that binds to BDCA2 according to any one of claims 1-5, wherein the method comprises the following steps:
a) culturing the host cell according to claim 9 under expression conditions to express the antibody or antigen-binding fragment thereof that binds to BDCA2;
b) isolating and purifying the antibody or antigen-binding fragment thereof that binds to BDCA2 described in step a).

11. A composition, wherein the composition comprises the antibody or antigen-binding fragment thereof that binds to BDCA2 according to any one of claims 1-5 and a pharmaceutically acceptable carrier.

12. Use of the antibody or antigen-binding fragment thereof that binds to BDCA2 according to any one of claims 1-5 or the composition according to claim 11 in the manufacture of a medicament for treating an inflammatory disease or autoimmune disease.

13. The use according to claim 12, wherein the inflammatory disease or autoimmune disease is selected from the group consisting of: systemic lupus erythematosus, cutaneous lupus erythematosus, discoid lupus, lupus nephritis, cutaneous lupus, rheumatoid arthritis, inflammatory bowel disease, systemic sclerosis (scleroderma), psoriasis, type I diabetes, dermatomyositis, and polymyositis.

14. An antibody-drug conjugate, wherein the antibody-drug conjugate comprises:
(a) an antibody moiety, the antibody moiety comprises the antibody or antigen-binding fragment thereof according to any one of claims 1-5; and
(b) a conjugate moiety conjugated to the antibody moiety, the conjugate moiety is selected from the group consisting of: a detectable label, a drug, a toxin, a cytokine, a radionuclide, an enzyme, or a combination thereof.

15. A CAR construct, wherein the scFv segment of the monoclonal antibody antigen-binding region of the CAR construct is a binding region that specifically binds to BDCA2, and the heavy chain variable region of the scFv comprises:
heavy chain complementarity determining regions H-CDR1, H-CDR2, and H-CDR3, wherein the amino acid sequence of H-CDR1 is as shown in SYIIH (SEQ ID NO: 6), the amino acid sequence of H-CDR2 is as shown in TIYPGNGDTSYNQKFKG (SEQ ID NO: 7), the amino acid sequence of H-CDR3 is as shown in MGDNEYFDY (SEQ ID NO: 8), and
the light chain variable region of the scFv comprises:
light chain complementarity determining regions L-CDR1, L-CDR2, and L-CDR3, wherein the amino acid sequence of the L-CDR1 is as shown in RASGNIHNYLA (SEQ ID NO: 9), the amino acid sequence of L-CDR2 is as shown in DAETLAD (SEQ ID NO: 10), the amino acid sequence of L-CDR3 is as shown in QHFWSTPYT (SEQ ID NO: 11);
wherein any one of the aforementioned amino acid sequences further optionally comprises a derivative sequence that optionally undergoes addition, deletion, modification, and/or substitution of at least one amino acid and is capable of retaining BDCA2 binding affinity.
